# EUROPEAN PATENT APPLICATION

(11) **EP 2 008 997 A1**
(43) Date of publication of application: **31.12.2008**
(21) Application number: 07736964.3
(22) Date of filing: 27.03.2007
(51) Int. Cl.: C07C 269/06, C07C 269/04, C07C 271/20, C07C 271/22, C07D 207/09, C07D 207/16, C07D 231/14, C07D 307/85, C07D 403/12, C07B 53/00, C07B 61/00

(54) **PROCESS FOR PRODUCTION OF ETHYLENEDIAMINE DERIVATIVE HAVING HALOGENATED CARBAMATE GROUP AND ACYL GROUP, AND INTERMEDIATED FOR PRODUCTION OF THE DERIVATIVE**

(30) Priority: 29.03.2006 JP 2006090705
(71) Applicant: Mitsui Chemicals, Inc., Tokyo 105-7117 (JP)
(72) Inventor: UMETANI, Hideki, Omuta-shi, Fukuoka 836-8610 (JP); KOHNO, Toshiyuki, Mobara-shi, Chiba 297-0017 (JP)
(74) Representative: Stuart, Ian Alexander
(86) International application number: PCT/JP2007/000307
(87) International publication number: WO 2007/111024

(57) **Abstract**

Ethylenedamine derivatives having a halogenated carbamate group and an acyl group can be produced by performing the catalytic hydrogenation of an aminonitrile having a halogen-substituted carbamate group in the presence of- an acid and then performing the acylation of the resulting product. The aminonitrile (i.e., a starting material) can be prepared in a high yield by performing the halogenated carbamatation of an amino acid amide in the presence of water and then reacting the resulting product with a deoxidizing agent such as a vilsmeier reagent.

## Description

### TECHNICAL FIELD

The field of the present invention relates to a process for producing ethylenediamine derivatives having a halogenated carbamate group and an acyl group, and intermediates for production of the derivatives.

### BACKGROUND ART

There has been known that ethylenediamine derivatives having a halogenated carbamate group and an acyl group is useful as a fungicide as shown in Patent Document 1. To prepare such a compound group, it is very important to produce ethylenediamine derivatives having a carbamate group that are intermediates for production of the compound thereof with good efficiency.

Examples of the conventional technique for production of ethylenediamine derivatives having a carbamate group include (I) a process including converting amino alcohols having a carbamate group into a phthalimide adduct using phthalimide, triphenylphosphine and diethylazodicarboxylate, and then subjecting the phthalimide adduct to deprotection with hydrazine (Non-patent Document 1), (II) a process including carrying out the catalytic hydrogenation of aminonitriles having a carbamate group in an ethanol solvent saturated with ammonia in the presence of Raney nickel (Non-patent Document 2) and the like.
Patent Document 1: WO2005042474
Non-patent Document 1: Tetrahedron Asymmetry, Vol. 11, pp. 1907 to 1910, 2000
Non-patent Document 2: Synthetic Communications, Vol. 24, No. 12, pp. 1767 to 1772, 1994

### DISCLOSURE OF THE INVENTION

Since the aforementioned process (I) as described in Non-patent Document 1 employs diethylazodicarboxylate or hydrazine which has a problem in that a plurality of and any amount of reaction by-products are produced and a problem in the safety, it is difficult to mention that it is suitable for an industrial process.
On the other hand, the aforementioned process (II) as described in Non-patent Document 2 is excellent in the yield, and also has an advantage in the industrial production process. However, when the aforementioned process (II) is applied, for example, to aminonitriles having a halogen-substituted carbamate group such as a 2,2,2-trifluoroethoxycarbonylaminonitrile derivative, it has been made clear, as a result of the review by the present inventors, that a compound obtained by converting a carbamate group into a ureido group is obtained even without progressing the intended reduction reaction.
In other words, since the halogen-substituted carbamate group has good reactivity as compared to usual hydrocarbon-based carbamate groups, it has turned out that it is extremely difficult to apply the prior art. It has become clear that the reactivity at this time becomes a problem in common when a compound group having a halogen-substituted carbamate group is produced as well as when the reduction reaction of nitrile is conducted. For that reason, a production progress with good efficiency needs to be developed for every production intermediates.

The present invention is to solve a novel object of effectively producing ethylenediamine derivatives having a halogenated carbamate group and an acyl group, and to provide a process which is favorable to the industrial production of the ethylenediamine derivatives.

The present inventors have conducted an extensive study and as a result, have found an effective solution to the above object by carrying out the catalytic hydrogenation of aminonitriles having a halogen-substituted carbamate group in the presence of an acid and then performing the acylation of the resulting product. Furthermore, we have found that the aminonitriles are prepared in a high yield by performing the halogenated carbamatation of amino acid amides in the presence of water and then reacting the resulting product with a deoxidizing agent such as a vilsmeier reagent. Thus, the present invention has been completed.

That is, the present invention is specified by matters described in below:
[1] a process for producing a compound represented by the general formula (4) comprising;
   converting the compound represented by the general formula (1) into a compound represented by the general formula (2) by performing the catalytic hydrogenation of a compound represented by the general formula (1) in the presence of an acid, and
   reacting the compound represented by the general formula (2) with a compound represented by the general formula (3),

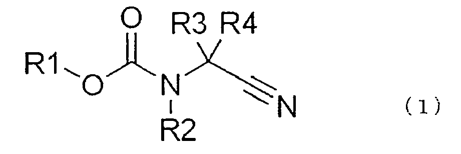

wherein, in the formula, R1 represents an alkyl group having 1 to 6 carbon atoms which is substituted with at least one halogen atom or a cycloalkyl group having 3 to 6 carbon atoms which is substituted with at least one halogen atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom),

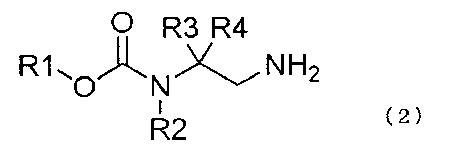

wherein, in the formula, R1, R2, R3 and R4 are the same as those described above,

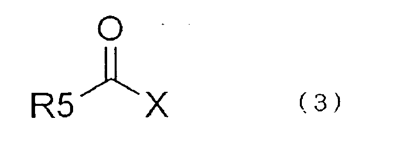

wherein, in the formula, R5 represents an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and X represents a leaving group,

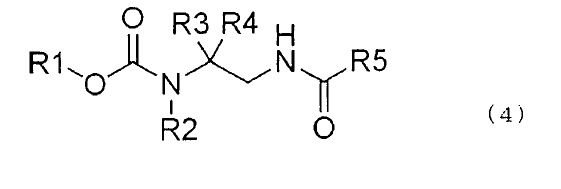

wherein, in the formula, R1, R2, R3, R4 and R5 are the same as those described above;
[2] the process for producing a compound represented by the general formula (4) as set forth in [1], in which the compound represented by the general formula (1) is obtained by reacting a compound represented by the general formula (5) with a deoxidizing agent,

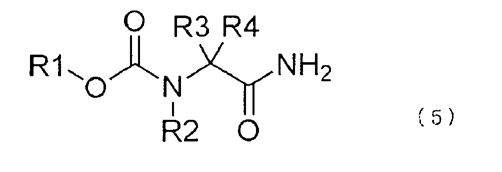

wherein, in the formula, R1, R2, R3 and R4 are the same as those described above;
[3] the process for producing a compound represented by the general formula (4) as set forth in [2], in which the compound represented by the general formula (5) is obtained by reacting a compound represented by the general formula (6) with a compound represented by the general formula (7) in the presence of water,

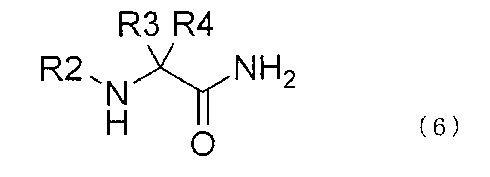

wherein, in the formula, R2, R3 and R4 are the same as those described above,

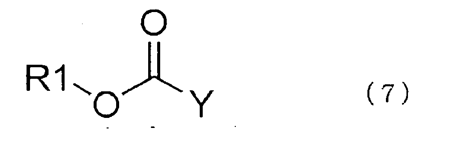

wherein, in the formula, R1 is the same as those described above; and Y represents a halogen atom;
[4] the process for producing a compound represented by the general formula (4) as set forth in [1], in which, in the formulas, R1 represents an alkyl group having 1 to 6 carbon atoms which is substituted with at least one halogen atom; R2 represents a hydrogen atom or an alkyl group having 1 to 6 carbon atoms; R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom); and R5 represents an aryl group which may be substituted or a heteroaryl group which may be substituted;
[5] the process for producing a compound represented by the general formula (4) as set forth in [2], in which, in the formulas, R1 represents an alkyl group having 1 to 6 carbon atoms which is substituted with at least one halogen atom; R2 represents a hydrogen atom or an alkyl group having 1 to 6 carbon atoms; R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom); and R5 represents an aryl group which may be substituted or a heteroaryl group which may be substituted;
[6] the process for producing a compound represented by the general formula (4) as set forth in [3], in which, in the formula, R1 represents an alkyl group having 1 to 6 carbon atoms which is substituted with at least one halogen atom; R2 represents a hydrogen atom or an alkyl group having 1 to 6 carbon atoms; R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom); and R5 represents an aryl group which may be substituted or a heteroaryl group which may be substituted;
[7] the process for producing a compound represented by the general formula (4) as set forth in [4], in which, in the formula, R1 is an alkyl group having 1 to 6 carbon atoms which is substituted with at least one fluorine atom;
[8] the process for producing a compound represented by the general formula (4) as set forth in [5], in which, in the formula, R1 is an alkyl group having 1 to 6 carbon atoms which is substituted with at least one fluorine atom;
[9] the process for producing a compound represented by the general formula (4) as set forth in [6], in which, in the formula, R1 is an alkyl group having 1 to 6 carbon atoms which is substituted with at least one fluorine atom;
[10] a process for producing a compound represented by the general formula (2) comprising;
   converting the compound represented by the general formula (1) into a compound represented by the general formula (2) by performing the catalytic hydrogenation of a compound represented by the general formula (1) in the presence of an acid,

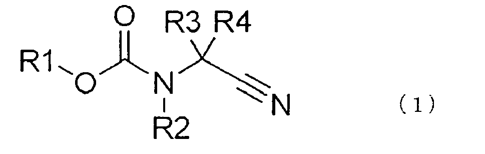

wherein, in the formula, R1, R2, R3 and R4 are the same as those described in [1],

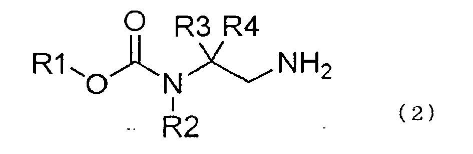

wherein, in the formula, R1, R2, R3 and R4 are the same as those described in [1];
[11] the process for producing a compound represented by the general formula (2) as set forth in [10], in which, in the formulas, R1 represents an alkyl group having 1 to 6 carbon atoms which is substituted with at least one halogen atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom);
[12] the process for producing a compound represented by the general formula (2) as set forth in [11], in which, in the formula, R1 is an alkyl group having 1 to 6 carbon atoms which is substituted with at least one fluorine atom;
[13] a process for producing a compound represented by the general formula (1) comprising;
   converting the resulting product into a compound represented by the general formula (1) by reacting a compound represented by the general formula (5) with a deoxidizing agent,

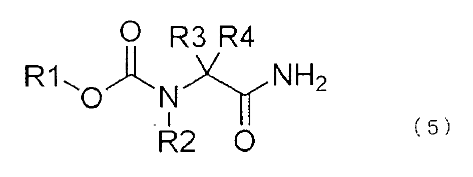

wherein, in the formula, R1, R2, R3 and R4 are the same as those described in [2],

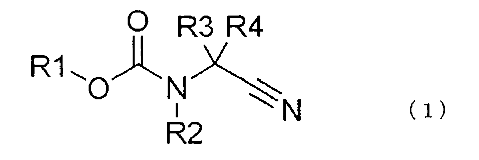

wherein, in the formula, R1, R2, R3 and R4 are the same as those described in [2];
[14] the process for producing a compound represented by the general formula (1) as set forth in [13], in which, in the formulas, R1 represents an alkyl group having 1 to 6 carbon atoms which is substituted with at least one halogen atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom);
[15] the process for producing a compound represented by the general formula (1) as set forth in [14], in which, in the formula, R1 is an alkyl group having 1 to 6 carbon atoms which is substituted with at least one fluorine atom;
[16] a process for producing a compound represented by the general formula (5) comprising;
   converting the resulting product into a compound represented by the general formula (5) by reacting a compound represented by the general formula (6) with a compound represented by the general formula (7) in the presence of water,

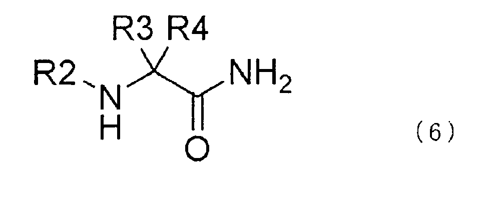

wherein, in the formula, R2, R3 and R4 are the same as those described in [3],

-

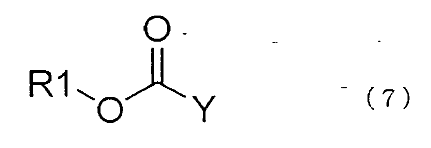

wherein, in the formula, R1 and Y are the same as those described in [3],

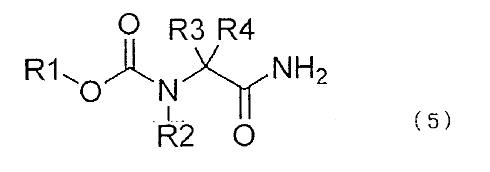

wherein, in the formula, R1, R2, R3 and R4 are the same as those described in [3];
[17] the process for producing a compound represented by the general formula (5) as set forth in [16], in which, in the formulas, R1 represents an alkyl group having 1 to 6 carbon atoms which is substituted with at least one halogen atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom);
[18] the process for producing a compound represented by the general formula (5) as set forth in [17], in which, in the formula, R1 is an alkyl group having 1 to 6 carbon atoms which is substituted with at least one fluorine atom;
[19] a compound represented by the general formula (2),

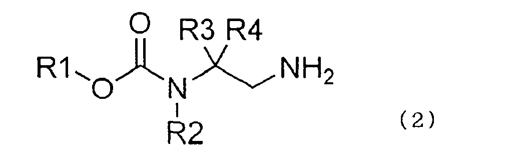

wherein, in the formula, R1 represents an alkyl group having 1 to 6 carbon atoms which is substituted with at least one fluorine atom or a cycloalkyl group having 3 to 6 carbon atoms which is substituted with at least one fluorine atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom);
[20] the compound represented by the general formula (2) as set forth in [19], wherein, in the formula, R1 represents an alkyl group having 1 to 6 carbon atoms which is substituted with at least one fluorine atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom);
[21] a compound represented by the general formula (1),

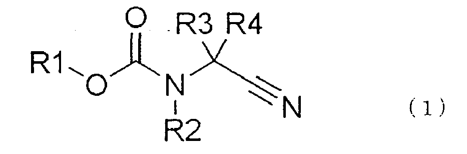

wherein, in the formula, R1 represents an alkyl group having 1 to 6 carbon atoms which is substituted with at least one fluorine atom or a cycloalkyl group having 3 to 6 carbon atoms which is substituted with at least one fluorine atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one-of R3 and R4 may be bonded with R2 to form a ring structure having 5 to 6 atoms in total (4 to 5 carbon atoms and 1 nitrogen atom);
[22] the compound represented by the general formula (1) as set forth in [21], wherein, in the formula, R1 represents an alkyl group having 1 to 6 carbon atoms which is substituted with at least one fluorine atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom);
[23] a compound represented by the general formula (5),

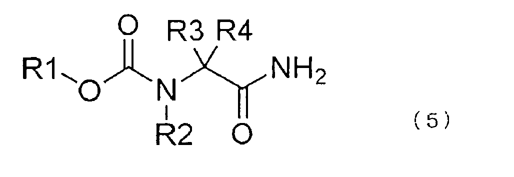

wherein, in the formula, R1 represents an alkyl group having 1 to 6 carbon atoms which is substituted with at least one fluorine atom or a cycloalkyl group having 3 to 6 carbon atoms which is substituted with at least one fluorine atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl-group having 3- to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom); and
[24] the compound represented by the general formula (5) as set forth in [23], wherein, in the formula, R1 represents an alkyl group having 1 to 6 carbon atoms which is substituted with at least one fluorine atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom).

According to the present invention, it is possible to provide a novel process for producing ethylenediamine derivatives having a halogenated carbamate group and an acyl group, and novel intermediates for production of the derivatives. Furthermore, the catalytic hydrogenation in the present invention has advantages such that it can be a reaction capable of recycling a catalyst, industrial wastes can be reduced, a reagent that is a problem in terms of safety can be omitted, and the derivatives can be produced in a high yield. For that reason, the present invention is excellent in environmental sustainability, economical efficiency, safety and productivity, and is useful as an industrial production process.

### BEST MODE FOR CARRYING OUT THE INVENTION

The present invention will be described in more detail below.
The present invention relates to a process for producing a compound represented by the general formula (4) comprising;
converting the compound represented by the general formula (1) into a compound represented by the following general formula (2) by performing the catalytic hydrogenation of a compound represented by the following general formula (1) in the presence of an acid, and
reacting the compound represented by the general formula (2) with a compound represented by the general formula (3).

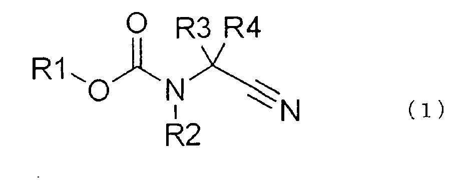

In the formula (1), R1 represents an alkyl group having 1 to 6 carbon atoms which is substituted with at least one halogen atom or a cycloalkyl group having 3 to 6 carbon atoms which is substituted with at least one halogen atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom).

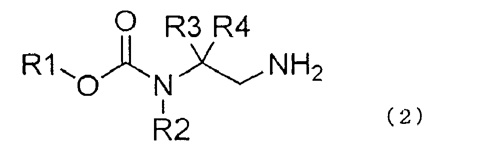

In the formula (2), R1, R2, R3 and R4 are the same as those described above.

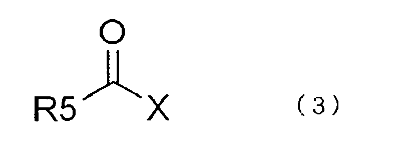

In the formula (3), R5 represents an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and X represents a leaving group.

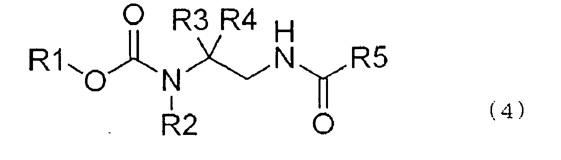

In the formula (4), R1, R2, R3, R4 and R5 are the same as those described above.
In the compound represented by the general formula (1), R1 represents an alkyl group having 1 to 6 carbon atoms which is substituted with at least one halogen atom or a cycloalkyl group having 3 to 6 carbon atoms which is substituted with at least one halogen atom.

The halogen atom in R1 of the general formula (1) represents fluorine, chlorine, bromine, iodine or the like.

The alkyl group having 1 to 6 carbon atoms in R1 of the general formula (1) represents a linear alkyl group such as a methyl group, an ethyl group, a propyl group, a butyl group, a pentyl group, a hexyl group or the like; or a branched alkyl group such as an isopropyl group, an isobutyl group, a sec-butyl group, a 1-methylbutyl group, a 2-methylbutyl group, a 3-methylbutyl group, a 1,1-dimethylpropyl group, a 2,2-dimethylpropyl group, a 1,2-dimethylpropyl group, a 1-methylpentyl group, a 2-methylpentyl group, a 3-methylpentyl group, a 4-methylpentyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group or the like. At least one halogen atom may be present in these alkyl groups. When two or more parts of alkyl groups are substituted with halogens, the halogens may be the same or different. There is no particular restriction in this regard.

The cycloalkyl group having 3 to 6 carbon atoms in R1 of the general formula (1) represents a cyclopropyl group, a cyclobutyl group, a cyclopentyl group, a cyclohexyl group or the like.

In the compound represented by the general formula (1), R2 represents a hydrogen atom, an alkyl group having 1 to 6 carbon atoms, a cycloalkyl group having 3 to 6 carbon atoms, an aryl group which may be substituted or a heteroaryl group which may be substituted.

The alkyl group having 1 to 6 carbon atoms in R2 of the general formula (1) is the same as those described in R1 of the general formula (1).

The cycloalkyl group having 3 to 6 carbon atoms in R2 of the general formula (1) is the same as those-described in R1 of the general formula (1).

Examples of the substituent in the aryl group which may be substituted or the heteroaryl group which may be substituted in R2 of the general formula (1) include alkyl groups such as a methyl group, an ethyl group, a propyl group, an isopropyl group, a butyl group, an isobutyl group, a sec-butyl group and the like; cycloalkyl groups such as a cyclopropyl group, a cyclobutyl group, a cyclopentyl group, a cyclohexyl group and the like; halogen-substituted alkyl groups such as a trifluoromethyl group, a difluoromethyl group, a bromodifluoromethyl group, a trifluoroethyl group and the like; alkoxy groups such as a methoxy group, an ethoxy group, a propoxy group, an isopropoxy group, a butoxy group, an isobutoxy group, a sec-butoxy group and the like; halogen-substituted alkoxy groups such as a trifluoromethoxy group, a difluoromethoxy group, a trifluoroethoxy group and the like; alkoxycarbonyl groups such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group and the like; aryloxycarbonyl groups such as a phenoxycarbonyl group and the like; alkylsulfonyl groups such as a methanesulfonyl group, an ethanesulfonyl group, a propanesulfonyl group, a butanesulfonyl group and the like; halogen-substituted alkylsulfonyl groups such as a trifluoromethanesulfonyl group, a difluoromethanesulfonyl group, a trifluoroethanesulfonyl group and the like; alkylcarbonyl groups such as a methylcarbonyl group, an ethylcarbonyl group, a propylcarbonyl group, an isopropylcarbonyl group and the like; cycloalkylcarbonyl groups such as a cyclopropylcarbonyl group, a cyclobutylcarbonyl group, a cyclopropylcarbonyl group, a cyclopentylcarbonyl group, a cyclohexylcarbonyl group and the like; arylcarbonyl groups such as a benzoyl group and the like; alkylcarbonyloxy groups such as a methylcarbonyloxy group, an ethylcarbonyloxy group, a propylcarbonyloxy group, an isopropylcarbonyloxy group and the like; cycloalkylcarbonyloxy groups such as a cyclopropylcarbonyloxy group, a cyclobutylcarbonyloxy group, a cyclopentylcarbonyloxy group, a cyclohexylcarbonyloxy group and the like; and arylcarbonyloxy groups such as a benzoyloxy group and the like. The number of substituents on the aryl group or the heteroaryl group is not restricted. Furthermore, when two or more parts of the aryl group or the heteroaryl group are substituted, the substituents may be the same or different. There is no restriction in this regard.

The aryl group in R2 of the general formula (1) represents a phenyl group, a naphthyl group, an anthranil group, a phenanthryl group or the like.

Examples of the heteroaryl group in R2 of the general formula (1) include nitrogen-containing heterocyclic groups such as a pyridyl group, a pyrimidyl group, a pyrazolyl group, a pyrazinyl group, a pyridazynyl group, an imidazolyl group, an indolyl group, a quinolyl group, a quinoxalyl group, a benzimidazolyl group and the like; oxygen-containing heterocyclic groups such as a tetrahydrofuranyl group, a furanyl group, a pyranyl group, a dioxanyl group, a 2,3-dihydrobenzo[1,4]dioxynyl group, a benzofuranyl group and the like; and heterocyclic groups containing two or more hetero atoms such as an oxazolyl group, an isoxazolyl group, a benzoxazolyl group, a benzoisoxazolyl group and the like.

In the compound represented by the general formula (1), R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted. Furthermore, R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom).

In R3 and R4 of the general formula (1), examples of the substituent in the alkyl group having 1 to 6 carbon atoms which may be substituted, the cycloalkyl group having 3 to 6 carbon atoms which may be substituted, the aryl group which may be substituted, the arylalkyl group which may be substituted, the heteroaryl group which may be substituted or the heteroarylalkyl group which may be substituted include alkyl groups such as a methyl group, an ethyl group, a propyl group, an isopropyl group, a butyl group, an isobutyl group, a sec-butyl group and the like; cycloalkyl groups such as a cyclopropyl group, a cyclobutyl group, a cyclopentyl group, a cyclohexyl group and the like; halogen-substituted alkyl groups such as a trifluoromethyl group, a difluoromethyl group, a bromodifluoromethyl group, a trifluoroethyl group and the like; alkoxy groups such as a methoxy group, an ethoxy group, a propoxy group, an isopropoxy group, a butoxy group, an isobutoxy group, a sec-butoxy group and the like; cycloalkoxy groups such as a cyclopropoxy group, a cyclobutoxy group, a cyclopentyloxy group, a cyclohexyloxy group and the like; halogen-substituted alkoxy groups such as a trifluoromethoxy group, a difluoromethoxy group, a trifluoroethoxy group and the like; alkoxycarbonyl groups such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group and the like; cycloalkoxycarbonyl groups such as a cyclopropoxycarbonyl group, a cyclobutoxycarbonyl group, a cyclopentyloxycarbonyl group, a cyclohexyloxycarbonyl group and the like; aryloxycarbonyl groups such as a phenoxycarbonyl group and the like; alkylsulfonyl groups such as a methanesulfonyl group, an ethanesulfonyl group, a propanesulfonyl group, a butanesulfonyl group and the like; halogen-substituted alkylsulfonyl groups such as a trifluoromethanesulfonyl group, a difluoromethanesulfonyl group, a trifluoroethanesulfonyl group and the like; alkylcarbonyl groups such as a methylcarbonyl group, an ethylcarbonyl group, a propylcarbonyl group, an isopropylcarbonyl group-and the like; cycloalkylcarbonyl groups such as a cyclopropylcarbonyl group, a cyclobutylcarbonyl group, a cyclopropylcarbonyl group, a cyclopentylcarbonyl group, a cyclohexylcarbonyl group and the like; arylcarbonyl groups such as a benzoyl group and the like; alkylcarbonyloxy groups such as a methylcarbonyloxy group, an ethylcarbonyloxy group, a propylcarbonyloxy group, an isopropylcarbonyloxy group and the like; cycloalkylcarbonyloxy groups such as a cyclopropylcarbonyloxy group, a cyclobutylcarbonyloxy group, a cyclopentylcarbonyloxy group, a cyclohexylcarbonyloxy group and the like; arylcarbonyloxy groups such as a benzoyloxy group and the like; and halogens such as fluorine, chlorine, bromine, iodine and the like. As for the alkyl group, the cycloalkyl group, the arylalkyl group, the heteroarylalkyl group, the aryl group or the heteroaryl group, the number of substituents is not restricted. As for the alkyl group, the cycloalkyl group, the arylalkyl group, the heteroarylalkyl group, the aryl group or the heteroaryl group, when two or more parts thereof are substituted, the substituents may be the same or different. There is no restriction in this regard. However, R3 or R4 that is a trifluoromethyl group is excluded, and halogen-substituted aromatic groups subjected to the catalytic hydrogenation, for example, halogenated aryl groups such as chlorophenyl, bromophenyl phenyl and the like, or halogenated heteroaryl groups such as chloropyridine and the like are also excluded.

The alkyl group having 1 to 6 carbon atoms in-R3 or R4 of the general formula (1) is the same as those described in R1 of the general formula (1).

The cycloalkyl group having 3 to 6 carbon atoms in R3 or R4 of the general formula (1) is the same as those described in R1 of the general formula (1).

The aryl group in R3 or R4 of the general formula (1) is the same as those described in R2 of the general formula (1).

In the arylalkyl group in R3 or R4 of the general formula (1), the aryl portion thereof is the-same as the aryl group described in R2 of the general formula (1), while the alkyl portion thereof represents an alkyl group having 1 to 4 carbon atoms.

Examples of the heteroaryl group in R3 or R4 of the general formula (1) include nitrogen-containing heterocyclic groups such as a pyridyl group, a pyrimidyl group, a pyrazolyl group, a pyrazinyl group, a pyridazynyl group, an imidazolyl group, an indolyl group, a quinolyl group, a quinoxalyl group, a benzimidazolyl group and the like; oxygen-containing heterocyclic groups such as a tetrahydrofuranyl group, a furanyl group, a pyranyl group, a dioxanyl group, a 2,3-dihydrobenzo[1,4]dioxynyl group, a benzofuranyl group and the like; and heterocyclic groups containing two or more hetero atoms such as an oxazolyl group, an isoxazolyl group, a benzoxazolyl group, a benzoisoxazolyl group and the like.

In the heteroarylalkyl group in R3 or R4 of the general formula (1), the heteroaryl portion thereof is the same as the heteroaryl group described in R2 of the general formula (1), while the alkyl portion thereof represents an alkyl group having 1 to 4 carbon atoms.

When the compound represented by the general formula (1) has an asymmetric carbon atom, an optically active substance or racemate can be used.

- It is possible to perform the catalytic hydrogenation of the compound represented by the general formula (1) in the presence of an acid for converting the compound-represented by the general formula (1) into the compound represented by the general formula (2).
Accordingly, generation of a by-product can be suppressed and the compound represented by the general formula (2) can be obtained in a high yield.

In the compound represented by the general formula (2), R1, R2, R3 and R4 are the same as those described in the general formula (1).

The acid in use is not restricted as long as it does not decompose the compound represented by the general formula (1) or (2). For example, organic acids or inorganic acids can be used.

Examples of the organic acid include formic acid, acetic acid, methanesulfonic acid and the like, while examples of the inorganic acid include hydrochloric acid, sulfuric acid, phosphoric acid and the like.

The amount of the acid used is not restricted as long as the intended reaction proceeds, but it is usually from not less than 1 equivalents to not more than 20 equivalents.

As a catalytic hydrogenation method, a method for carrying out the catalytic hydrogenation with metals such as palladium, platinum, rhodium, ruthenium and the like can be cited. These metals can be used in the form of a metal oxide, a metal chloride or the like.

The amount of the metal to be used for performing the catalytic hydrogenation method is not particularly limited as long as the reaction proceeds, but it is preferably equal to or not more than the weight of the general formula (1) from the economic perspectives.

As the type of the metal in use, metals supported in an activated carbon, SiO₂, Al₂O₃, BaSO₄, TiO₂, ZrO₂, MgO, ThO₂, diatomaceous earth or the like can be used. Regardless of its type, though, it is preferable to use a carrier which can be recycled from the economic perspectives.

A solvent to be used for carrying out the catalytic hydrogenation method is not particularly limited as long as the reaction proceeds. Concrete examples thereof include alcohol solvents such as methanol, ethanol, isopropanol and the like; aromatic solvents such as benzene, toluene, xylene and the like; hydrocarbon solvents such as hexane, heptane and the like; amide solvents such as dimethylformamide, dimethylacetamide, 1-methyl-2-pyrrolidone and the like; ether solvents such as diethyl ether, diisopropyl ether, 1,2-dimethoxyethane, tetrahydrofuran, dioxane and the like; ester solvents such as ethyl acetate, butyl acetate, isopropyl acetate and the like; and water. These solvents can be used singly, or two or more kinds thereof can be used in combination at an optional ratio.

The amount of the solvent used is not particularly limited, but it is usually preferably from 3 to 40 times weight, based on the weight of the general formula (1).

The reaction type is not particularly limited, but it is preferable that the compound presented in general formula (1) or the compound presented in general formula (1) diluted with the above solvent is added dropwise to a solvent containing a metal and an acid in the presence of a hydrogen source.

The reaction temperature is not particularly limited as long as the compound is not decomposed, but it is usually from not less than -10 to not more than 150 degree centigrade or not more than the boiling point of a solvent.

The reaction pressure is not particularly limited, and the reaction may be carried out in an atmospheric pressure or an applied pressure.

The hydrogen source to be used for the catalytic hydrogenation is not particularly limited as long as the reaction proceeds, but a transfer hydrogenation using cyclohexene, formic acid, formate and the like in addition to hydrogen gas can be used.

The equivalent of cyclohexene, formic acid and formate to be used for carrying out the reaction by the transfer hydrogenation is not particularly limited as long as the amount of generated hydrogen is to be not less than 2 equivalents, but it is preferably from not less than 2 equivalents to not more than 10 equivalents from the economic perspectives.

The usage of the compound represented by the general formula (2) obtained by the above reaction in the next step is not particularly limited. The reaction solution containing the compound represented by the general formula (2) can be subjected to a usual post-treatment such as removal of a solvent, liquid separation or the like, and then used in the next step without performing isolation and purification, or those in the form of a salt with an inorganic acid such as hydrochloric acid, sulfuric acid, phosphoric acid or the like, or an organic acid such as oxalic acid, fumaric acid, maleic acid, formic acid, acetic acid, methanesulfonic acid or the like can be used in the next step.

The compound represented by the general formula (2) also contains a salt formed with an inorganic acid or an organic acid. Examples of the inorganic acid include hydrochloric acid, sulfuric acid, phosphoric acid and the like, while examples of the organic acid include oxalic acid, fumaric acid, maleic acid, formic acid, acetic acid, methanesulfonic acid and the like.

By reacting the compound represented by the general formula (2) with the compound represented by the general formula (3), the resulting product can be converted into the compound represented by the general formula (4).

In the compound represented by the general formula (3), R5 represents an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted.

In R5, examples of the substituent in the alkyl group having 1 to 6 carbon atoms which may be substituted, the cycloalkyl group having 3 to 6 carbon atoms which may be substituted, the arylalkyl group which may be substituted, the heteroarylalkyl group which may be substituted, the aryl group which may be substituted or the heteroaryl group which may be substituted include alkyl groups such as a methyl group, an ethyl group, a propyl group, an isopropyl group, a butyl group, an isobutyl group, a sec-butyl group and the like; cycloalkyl groups such as a cyclopropyl group, a cyclobutyl group, a cyclopentyl group, a cyclohexyl group and the like; halogen-substituted alkyl groups such as a trifluoromethyl group, a difluoromethyl group, a bromodifluoromethyl group, a trifluoroethyl group and the like; alkoxy groups such as a methoxy group, an ethoxy group, a propoxy group, an isopropoxy group, a butoxy group, an isobutoxy group, a sec-butoxy group and the like; cycloalkoxy groups such as a cyclopropoxy group, a cyclobutoxy group, a cyclopentyloxy group; a cyclohexyloxy group and the like; halogen-substituted alkoxy groups such as a trifluoromethoxy group, a difluoromethoxy group, a trifluoroethoxy group and the like; alkoxycarbonyl groups such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group and the like; cycloalkoxycarbonyl groups such as a cyclopropoxycarbonyl group, a cyclobutoxycarbonyl group, a cyclopentyloxycarbonyl group, a cyclohexyloxycarbonyl group and the like; aryloxycarbonyl groups such as a phenoxycarbonyl group and the like; alkylthio groups such as a methylthio group, an ethylthio group, a propylthio group, a butylthio group and the like; halogen-substituted alkylthio groups such as a trifluoromethylthio group, a difluoromethylthio group, a trifluoroethylthio group and the like; alkylsulfinyl groups such as a methanesulfinyl group, an ethanesulfinyl group, a propanesulfinyl group, a butanesulfinyl group and the like; halogen-substituted alkylsulfinyl groups such as a trifluoromethanesulfinyl group, a difluoromethanesulfinyl group, a trifluoroethanesulfinyl group and the like; alkylsulfonyl groups such as a methanesulfonyl group, an ethanesulfonyl group, a propanesulfonyl group, a butanesulfonyl group and the like; halogen-substituted alkylsulfonyl groups such as a trifluoromethanesulfonyl group, a difluoromethanesulfonyl group, a trifluoroethanesulfonyl group and the like; alkylcarbonyl groups such as a methylcarbonyl group, an ethylcarbonyl group, a propylcarbonyl group, an isopropylcarbonyl group and the like; cycloalkylcarbonyl groups such as a cyclopropylcarbonyl group, a cyclobutylcarbonyl group, a cyclopropylcarbonyl group, a cyclopentylcarbonyl group, a cyclohexylcarbonyl group and the like; arylcarbonyl groups such as a benzoyl group and the like; alkylcarbonyloxy groups such as a methylcarbonyloxy group, an ethylcarbonyloxy group, a propylcarbonyloxy group, an isopropylcarbonyloxy group and the like; cycloalkylcarbonyloxy groups such as a cyclopropylcarbonyloxy group, a cyclobutylcarbonyloxy group, a cyclopentylcarbonyloxy group, a cyclohexylcarbonyloxy group and the like; arylcarbonyloxy groups such as a benzoyloxy group and the like; and halogen atoms such as chlorine, fluorine, bromine, iodine and the like. The number of substituents on the aryl group or the heteroaryl group is not restricted. Furthermore, when two or more parts of the aryl group or heteroaryl group are substituted, the substituents.may be the same or different. There is no restriction in this regard.

The alkyl group having 1 to 6 carbon atoms in R5 of the general formula (3) is the same as those described in R1 of the general formula (1).

The cycloalkyl group having 3 to 6 carbon atoms in R5 of the general formula (3) is the same as those described in R1 of the general formula (1).

The aryl group in R5 of the general formula (3) is the same as those described in R2 of the general formula (1).

In the arylalkyl group in R5 of the general formula (3), the aryl portion thereof is the same as the aryl group described in R2 of the general formula (1), while the alkyl portion thereof represents an alkyl group having 1 to 4 carbon atoms.

Examples of the heteroaryl group in R5 of the general formula (3) include nitrogen-containing heterocyclic groups such as a pyridyl group, a pyrimidyl group, a pyrazolyl group, a pyrazinyl group, a pyridazynyl group, an imidazolyl group, an indolyl group, a quinolyl group, a quinoxalyl group, a benzimidazolyl group and the like; sulfur-containing heterocyclic groups such as a tetrahydrothienyl group, a thienyl group, a thiopyranyl group, a benzothienyl group and the like; oxygen-containing heterocyclic groups such as a tetrahydrofuranyl group, a furanyl group, a pyranyl group, a dioxanyl group, a 2,3-dihydrobenzo[1,4]dioxynyl group, a benzofuranyl group and the like; and heterocyclic groups containing two or more hetero atoms such as an oxazolyl group, an isoxazolyl group, a thiazolyl group, an isothiazolyl group, a benzoxazolyl group, a benzoisoxazolyl group, a benzothiazolyl group, a benzoisothiazolyl group and the like.

In the heteroarylalkyl group in R5 of the general formula (3), the heteroaryl portion thereof is the same as the heteroaryl group in R5 of the general formula (3), while the alkyl portion thereof represents an alkyl group having 1 to 4 carbon atoms.
In the compound represented by the general formula (3), X represents a leaving group.

Examples of the leaving group represented by X of the general formula (3) include halogen atoms such as fluorine, chlorine, bromine, iodine and the like; alkoxy groups such as a methoxy group, an ethoxy group and the like; aryloxy groups such as a phenoxy group, a 4-nitrophenyl group and the like; acyloxy groups such as an acetyloxy group, a benzoyloxy group and the like; alkoxycarbonyloxy groups such as a methoxycarbonyloxy group, an ethoxycarbonyloxy group, an isobutyloxycarbonyloxy group and the like; arylcarbonyloxy groups such as a phenylcarbonyloxy group and the like; alkylthio groups such as a methylthio group and the like; a 2,5-dioxopyrrolidinyloxy group, a benzotriazolyloxy group, an imidazolyl group and the like.

In the compound represented by the general formula (4), R1, R2, R3 and R4 are the same as those described in the general formula (1), while R5 is the same as those described in the general formula (3).

The amount of the compound represented by the general formula (3) used is not particularly limited as long as it is equal to or not less than the equivalent of the compound represented by the general formula (2), but it is preferably from not less than 1 equivalent to not more than 3 equivalents from the economic perspectives.

When the compound represented by the general formula (2) forms a salt with an acid, or when an acid is generated while the compound represented by the general formula (2) is reacted with the compound represented by the general formula (3), a base can be used.

Examples of the base to be used include inorganic bases such as sodium hydroxide, potassium hydroxide, sodium hydrogen carbonate, potassium hydrogen carbonate, sodium carbonate, potassium carbonate and the like; and organic bases such as pyridine, collidine, picoline, 4-dimethylaminopyridine, lutidine, triethylamine, diisopropylamine, diisopropylethylamine, tributylamine, 1,8-diazabicyclo[5,4,0]-undec-7-ene, 1,4-diazabicyclo[2,2,0]octane, imidazole and the like. These bases can be used singly, or two or more kinds thereof can be used in combination at an optional ratio.

The base can be used in an amount of not less than 1 equivalent, based on the acid when the compound represented by the general formula (2) forms a salt with an acid. Or, when an acid is generated during the reaction, the base can be used in an amount of not less than 1 equivalent, based on the acid to be generated. Its upper limit is preferably not more than 10 equivalents from the economic perspectives.

The solvent to be used when the compound represented by the general formula (2) is reacted with the compound represented by the general formula (3) is not particularly limited as long as the compound represented by the general formula (4) is generated. Concrete examples of the solvent include halogen solvents such as dichloromethane, chloroform and the like; aromatic solvents such as benzene, toluene, xylene and the like; hydrocarbon solvents such as hexane, heptane and the like; amide solvents such as dimethylformamide, dimethylacetamide, 1-methyl-2-pyrrolidone and the like; urea solvents such as 1,3-dimethyl-2-imidazolidinone, 1,3-dimethyl-3,4,5,6-tetrahydro-2(1H)-pyrrolidinone and the like; ester solvents such as ethyl acetate, butyl acetate, isopropyl acetate and the like; ether solvents such as diethyl ether, diisopropyl ether, 1,2-dimethoxyethane, tetrahydrofuran, dioxane and the like; nitrile solvents such as acetonitrile, propionitrile and the like; alcohol solvents such as isopropanol, t-butyl alcohol and the like; and water. These solvents can be used singly, or two or more kinds thereof can be used in combination at an optional ratio.

The amount of the solvent used is not particularly limited, but it is usually from not less than 3 times weight to not more than 40 times weight, based on the compound represented by the general formula (2).

The reaction temperature when the compound represented by the general formula (2) is reacted with the compound represented by the general formula (3) is not particularly limited as long as the compound is not decomposed, but it is usually from -10 to 150 degree centigrade or not more than the boiling point of a solvent.

The way of obtaining the compound represented by the general formula (1) is not particularly limited, and commercial products may be purchased. Or there may be used the compound represented by the general formula (1) which is prepared by reacting the compound represented by the general formula (8) with the compound represented by the general formula (7) to be obtained with reference to the Strecker reaction exemplified in Japanese Patent Laid-open No. 2002-34593, Tetrahedron : Asymmetry, Vol. 12, pp. 219 to 228, 2001, Journal of American Chemical Society, Vol. 124, No. 34, pp. 10012 to 10014, 2002 or the like.

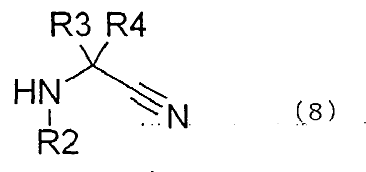

In the general formula (8), R2, R3 and R4 are the same as those described in the general formula (1).

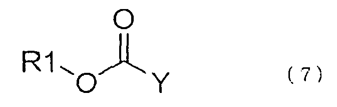

In the general formula (7), R1 is the same as those described in the general formula (1); and Y represents a halogen atom such as fluorine, chlorine, bromine, iodine or the like.

In addition, the compound represented by the general formula (1) can be prepared by reacting the compound represented by the general formula (5) with a deoxidizing agent,

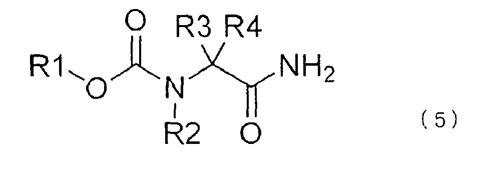

The reaction of the compound represented by the general formula (5) with a deoxidizing agent will be described below.
In the compound represented by the general formula (5), R1, R2, R3 and R4 are the same as those described in the general formula (1).

Examples of the deoxidizing agent include halogenating agents such as thionyl chloride, oxalyl chloride, phosgene, phosphorus oxychloride, phosphorus trichloride, phosphorus pentachloride, thionyl bromide, phosphorus tribromide, mesyl chloride, tosyl chloride and the like; carbodiimide derivatives such as N,N'-dicyclohexylcarbodiimide, N,N'-diisopropylcarbodiimide, 1-ethyl-3-(3-dimethylaminopropyl)carbodiimide hydrochloride and the like; anhydrides such as acetic anhydride, trifluoroacetic anhydride and the like; a vilsmeier reagent and the like.

The vilsmeier reagent refers to a compound represented by the general formula (9) which is prepared from formamide derivatives such as dimethylformamide or the like and a halogenating agent,

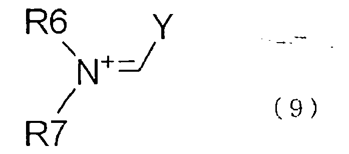

In the general formula (9), R6 and R7 each independently represent an alkyl group having 1 to 3 carbon atoms; and Y represents a halogen atom.
The compound represented by the general formula (9) also contains a salt derived from the halogenating agent.

The alkyl group having 1 to 3 carbon atoms in R6 and R7 of the general formula (9) represents a methyl group, an ethyl group, a propyl group or the like.

The halogen atom in Y of the general formula (9) is fluorine, chlorine, bromine, iodine or the like.

The usage of the deoxidizing agent is not particularly limited. Either of a method including adding a substrate to a deoxidizing agent or a method including adding a deoxidizing agent to a substrate may be used.

The usage of the deoxidizing agent when it is a vilsmeier reagent is not particularly limited either. A method including previously preparing a vilsmeier reagent in a solvent, and then adding the compound represented by the general formula (5) thereto, or a method including introducing the compound represented by the general formula (5) and a halogenating agent into a solvent containing formamide derivatives can be performed.

The amount of the deoxidizing agent used is not particularly limited as long as it is not less than 1 equivalent, based on the compound represented by t-he general formula (5), but it is usually from not less than 1 equivalent to not more than 10 equivalents.

The amount of the deoxidizing agent used when it is a vilsmeier reagent is not particularly limited as long as the halogenating agent is used in an amount of not less than 1 equivalent based on the compound represented by the general formula (5), and the formamide derivatives are used in an amount of not less than a catalytic amount. The amount of the halogenating agent is usually from not less than 1 equivalent to not more than 10 equivalents, while the amount of the formamide derivatives are usually from not less than 0.1 equivalent to not more than 10 equivalents based on the compound represented by the general formula (5). Furthermore, the formamide derivatives can also be used as a solvent.

The solvent to be used when the compound represented by the general formula (5) is converted into the compound represented by the general formula (1) is not particularly limited as long as it is an aprotic solvent. Concrete examples thereof include halogenated solvents such as dichloromethane, chloroform and the like; aromatic solvents such as benzene, toluene, xylene and the like; hydrocarbon solvents such as hexane, heptane and the like; amide solvents such as dimethylformamide, dimethylacetamide, 1-methyl-2-pyrrolidone and the like; ether solvents such as diethyl ether, diisopropyl ether, 1,2-dimethoxyethane, tetrahydrofuran, dioxane and the like; nitrile solvents such as acetonitrile, propionitrile and the like; urea solvents such as 1,3-dimethyl-2-imidazolidinone, 1,3-dimethyl-3,4,5,6-tetrahydro-2(1H)-pyrrolidinone and the like; and ester solvents such as ethyl acetate, butyl acetate, isopropyl acetate and the like. The solvents can be used singly, or two or more solvents can be used in combination at an optional ratio.

In the deoxidizing agents to be used for the present invention, a vilsmeier reagent can be preferably applied.

The amount of the solvent used is not particularly limited, but it is usually preferably from 3 to 40 times weight, based on the weight of the compound represented by the general formula (5).

The reaction temperature when the compound represented by the general formula (5) is converted into the compound represented by the general formula (1) is not particularly limited as long as the reaction proceeds, but it is from not less than -10 degree centigrade to not more than 150 degree centigrade or not more than the boiling point of a solvent.
Due to such a convenient reaction, the compound represented by the general formula (1) can be obtained in a high yield. For that reason, the reaction is useful as a process for industrially producing the compound represented by the general formula (1).

In the present invention, the aforementioned compound represented by the general formula (5) can be obtained by reacting a compound represented by the following general formula (6) with a compound represented by the following general formula (7) in the presence of water,

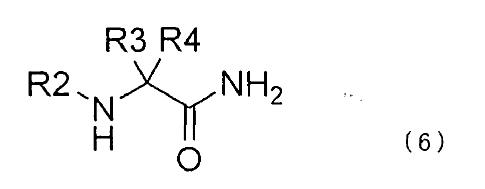

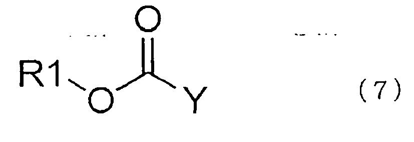

A process for producing the compound represented by the general formula (5) will be explained below.
In the general formula (6), R2, R3 and R4 are the same as those described in the general formula (1).

The compound represented by the general formula (6) also contains a salt formed with an inorganic acid or an organic acid. Examples of the inorganic acid include hydrochloric acid, sulfuric acid, phosphoric acid and the like, while examples of the organic acid include oxalic acid, fumaric acid, maleic acid, formic acid, acetic acid, methanesulfonic acid and the like.

Commercial products can be used for the general formula (6). At this time, a free body or a salt thereof may be used.

R1 in the general formula (7) is the same as R1 in the general formula (1), while Y is a halogen atom such as fluorine, chlorine, bromine, iodine or the like.

As a process for production of the compound represented by the general formula (5), the compound can be obtained with good efficiency by reacting the compound represented by the general formula (6) with the compound represented by the general formula (7) in the presence of water. The reaction carried out in the presence of water is a characteristic of the present invention. Because of this characteristic, the yield of the compound represented by the general formula (5) is remarkably improved.

The amount of the compound represented by the general formula (7) used is not particularly limited as long as it is not less than 1 equivalent, based on the compound represented by the general formula (6), but it is from not less than 1 equivalent to not more than 5 equivalents from the economic perspectives.

When the compound represented by the general formula (6) is reacted with the compound represented by the general formula (7), a base may be used. Examples of the base include inorganic bases such as sodium hydroxide, potassium hydroxide, sodium hydrogen carbonate, potassium hydrogen carbonate, sodium carbonate, potassium carbonate and the like; and organic bases such as pyridine, collidine, picoline, 4-dimethylaminopyridine, lutidine, triethylamine, diisopropylamine, diisopropylethylamine, tributylamine, 1,8-diazabicyclo[5,4,0]-undec-7-ene, 1, 4-diazabicyclo[2,2,0]octane and the like. The bases can be used singly, or two or more kinds thereof can be used in combination at an optional ratio.

The amount of the base used is not less than 1 equivalent, based on the compound represented by the general formula (6), or it is not particularly limited as long as it is not less than 2 equivalents when the compound represented by the general formula (6) is a salt. Its upper limit is preferably not more than 10 equivalents from the economic perspectives.

The solvent to be used when the compound represented by the general formula (6) is reacted with the compound represented by the general formula (7) is water or a solvent containing water. Two or more solvents containing water can be used in combination at an optional ratio. Concrete examples of the solvent to be mixed with water include halogenated solvents such as dichloromethane, chloroform and the like; aromatic solvents such as benzene, toluene, xylene and the like; hydrocarbon solvents such as hexane, heptane and the like; amide solvents such as dimethylformamide, dimethylacetamide, 1-methyl-2-pyrrolidone and the like; urea solvents such as 1,3-dimethyl-2-imidazolidinone, 1,3-dimethyl-3,4,5,6-tetrahydro-2(1H)-pyrrolidinone and the like; ester solvents such as ethyl acetate, butyl acetate, isopropyl acetate and the like; ether solvents such as diethyl ether, diisopropyl ether, 1,2-dimethoxyethane, tetrahydrofuran, dioxane and the like; and nitrile solvents such as acetonitrile, propionitrile and the like.

The compound represented by the general formula (7) can be diluted with a solvent and added dropwise. The solvent to be used at this time is not restricted as long as it is not reacted with the compound represented by the general formula (7). Concrete examples of the solvent to be diluted include halogenated solvents such as dichloromethane, chloroform and the like; aromatic solvents such as benzene, toluene, xylene and the like; hydrocarbon solvents such as hexane, heptane and the like; amide solvents such as dimethylformamide, dimethylacetamide, 1-methyl-2-pyrrolidone and the like; urea solvents such as 1, 3-dimethyl-2-imidazolidinone, 1,3-dimethyl-3,4,5,6-tetrahydro-2(1H)-pyrrolidinone and the like; ester solvents such as ethyl acetate, butyl acetate, isopropyl acetate and the like; ether solvents such as diethyl ether, diisopropyl ether, 1,2-dimethoxyethane, tetrahydrofuran, dioxane and the like; and nitrile solvents such as acetonitrile, propionitrile and the like.

The amount of the solvent used is not particularly limited, but it is usually preferably from 3 to 40 times weight, based on the weight of the compound represented by the general formula (6).

The reaction temperature is not particularly limited as long as the compound is not decomposed, but it is usually from not less than -30 degree centigrade to not more than 150 degree centigrade or not more than the boiling point of a solvent.

As described above, the compound represented by the general formula (4), i.e., ethylenediamine derivatives having a halogenated carbamate group and an acyl group can be effectively produced.

### EXAMPLES

The present invention is now illustrated in detail below with reference to Examples. However, the present invention is not restricted to these Examples.

Hereinafter, tetrahydrofuran is referred to as THF; diisopropyl ether as IPE; dimethylformamide as DMF; and isopropyl alcohol as IPA.

### Example 1

### Synthesis of N-(2,2,2-trifluoroethoxycarbonyl)-L-valinamide (hereinafter referred to as the compound (I))

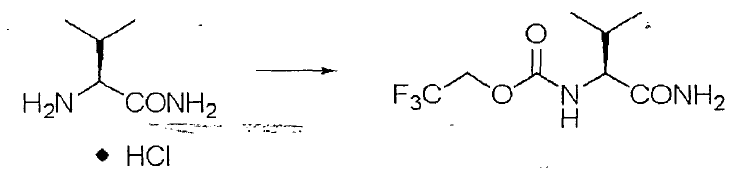

To 25.0 g of valinamide hydrochloride was added 325 ml of water, and the pH of the reaction solution became 8 by adding of a 8 weight % aqueous sodium hydroxide solution. 35 ml of dioxane containing 31.94 g of 2,2,2-trifluoroethoxycarbonyl chloride and a 8 weight % aqueous sodium hydroxide solution were added dropwise to the solution at the same time while maintaining the pH at 8 ± 0.5 at room temperature. After the completion of dropwise addition, the solution was stirred for 2 hours and then the precipitate was filtered and vacuum dried. The obtained compound of a white solid was the title compound.
Quantity: 37.80 g (Yield: 95%)
¹H NMR(DMSO-d₆)
δ0.89(3H,d,J=6.83Hz),0.86(3H,d,J=6.83Hz),1.98(1H,m),3.78(1H,dd ,J=6.83,8.78Hz),4.64(2H,m),7.05(1H,brs),7.37(1H,brs),7.61(1H,d ,J=8.78Hz).

### Comparative Example 1

### Synthesis of N-(2,2,2-trifluoroethoxycarbonyl)-L-valinamide without water

To 30 ml of a THF solution containing 3.0 g of valinamide hydrochloride was added 5.25 ml of pyridine, and then 5 ml of THF containing 3.83 g of 2,2,2-trifluoroethoxycarbonyl chloride was added dropwise to the solution . The resulting mixture was stirred at room temperature for 3 hours, and then water and ethyl acetate were added to the mixture ,and carried out liquid separation. The organic layer was washed with 1N hydrochloric acid, a saturated sodium hydrogen carbonate solution and a saturated sodium chloride solution, and then dried over sodium sulfate. After removing sodium sulfate, IPE was added and the resulting mixture was stirred. The obtained white precipitate was the compound (I), while the yield was 47% (quantity: 2.25 g). Even when the same reaction was carried out using triethylamine instead of pyridine, the yield was 30%. From these reaction results, it was determined that, in Example 1 carried out with an aqueous solvent, the reaction yield was remarkably improved.

### Example 2

### Synthesis of N-(2,2,2-trifluoroethoxycarbonyl)-L-valinonitrile (hereinafter referred to as the compound (II))

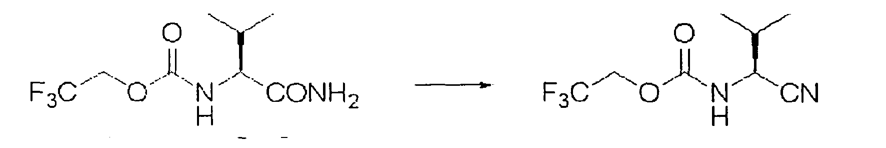

To 350 ml of toluene were added 35.0 g of the compound (I) and 35 ml of DMF, and the resulting mixture was stirred at room temperature. 35 ml of toluene containing 22.01 g of oxalyl chloride was added dropwise to the suspension with care. The solution was stirred at the same temperature for 2 hours, and then 350 ml of water was added to the mixture, and carried out liquid separation. Further, the separated organic layer was washed with 350 ml of water, and then the solvent was distilled off under reduced pressure. Next, by distillation, the fraction of distillate was isolated at 116 to 122 degree centigrade in 0.3 mmHg. The obtained colorless and transparent oily substance was the title compound.
Quantity: 29.89 g (Yield: 92%)
¹H NMR(CDCl₃)
δ1.10(3H,d,J=6.83Hz),1.12(3H,d,J=6.83Hz),2.09(1H,sept,J=6.83Hz ),4.4-4.6(3H,m),5.31(1H,brd).

### Example 3

### Synthesis of Compound (II) by Process for Production of Vilsmeier in Advance.

To 5 ml of toluene containing 1 ml of DMF was added dropwise 5 ml of a toluene solution containing 433 µl of oxalyl chloride at room temperature. The resulting solution was stirred for 30 minutes, and then 1.0 g of the compound (I) was introduced thereinto and reacted for 3 hours. The organic layer was washed with water, and then purified by silica gel chromatography to obtain a compound (II). -
Quantity: 0.92 g (Yield: > 99%)

### Example 4

### Synthesis of Compound (II) with Ethyl Acetate as Solvent

The synthesis was carried out in the same manner as in Example 2, except that 5.0 g of the compound (I) was used, a solvent was changed from toluene to ethyl acetate, and a purification method was changed from distillation to column chromatography. As a result, a compound (II) could be obtained.
Quantity: 4.45 g (Yield: 96%)

### Example 5

### Synthesis of (2S)-3-Methyl-N²-(2,2,2-trifluoroethoxycarbonyl)-butane-1,2-dia mine hydrochloride (hereinafter referred to as the compound (III)) (1)

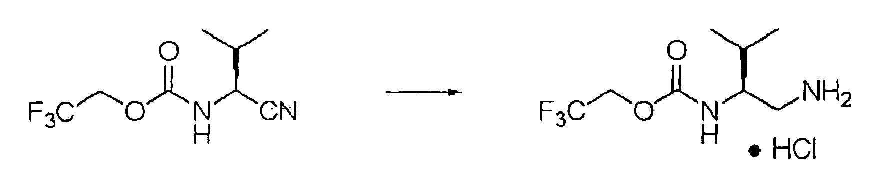

To 180 ml of IPA were successively added 26.8 g of acetic acid, 2.0 g of 5% palladium carbon (water: 49.5%, a product of N.E. Chem.) and 14.1 g of ammonium formate, and the resulting solution was sufficiently stirred. 10 ml of IPA containing 10.0 g of the compound (II) was added dropwise to the solution at room temperature, and then the solution was stirred at the same temperature for 2.5 hours. The catalyst was removed by filtering and then the solvent was distilled off under reduced pressure. To the residue were added water and ethyl acetate. Subsequently, potassium carbonate was added until the pH of the water layer became about 10 to carry out liquid separation. To the separated organic layer was added sodium sulfate, and the organic layer was dried and filtered. Thereafter, a 4N hydrogen chloride-ethyl acetate solution was added to the solution. When the solution was concentrated under reduced pressure, a white solid was precipitated and the precipitate was filtered to obtain the title compound.
Quantity: 10.5 g (Yield: 89%)
¹H NMR(DMSO-d₆)
δ0.83(3H,d,J=6.83Hz),0.85(3H,d,J=6.83Hz),1.77(1H,sept,J=6.83Hz ),2.74(1H,dd,J=9.76,13.17Hz),2.93(1H,dd,J=3.42,13.17Hz),3.54(1 H,m),4.55(1H,m),4.73(1H,m),7.67(1H,d,J=9.27Hz),8.02(3H,brs).

### Example 6

### Synthesis of Compound (III) (2)

In an autoclave, 50 ml of IPA containing 3.30 g of the compound (II), 9.0 g of acetic acid and 0.6 g of 5% palladium carbon (water: =49.5%, a product of N.E. Chem) was pressurized with hydrogen gas to 2.1 MPa, and then the reaction was carried out at room temperature. After the reaction was carried out for 5 hours, and then the catalyst was removed and the resulting solution was concentrated under reduced pressure. At this point of time, when a free body of the compound (III) was quantitatively analyzed with high performance liquid chromatography, the reaction yield was 95%. To the residue were added water and ethyl acetate, and subsequently the pH of the water layer was set to 10.7 by adding of a 8 weight % aqueous sodium hydroxide solution to carry out liquid separation. The organic layer was dried over sodium sulfate and filtered, and then 10 ml of a 4N hydrogen chloride-ethyl acetate solution was added to the solution. When the solution was concentrated under reduced pressure, a white solid was precipitated and the precipitate was filtered to obtain the title compound.
Quantity: 3.60 g (Yield: 92%)

### Example 7

### Synthesis of Compound (III) (3)

The reaction was carried out in the same manner as in Example 6, except that methanol was used instead of IPA and a catalytic amount was 5 times. A free body of the compound (III) was obtained with the reaction yield of 93%.

### Example 8

### Synthesis of Compound (III) (4)

The reaction was carried out in the same manner as in Example 7, except that ethanol was used instead of methanol. A free body of the compound (III) was obtained with the reaction yield of 91%.

### Reference Example 1

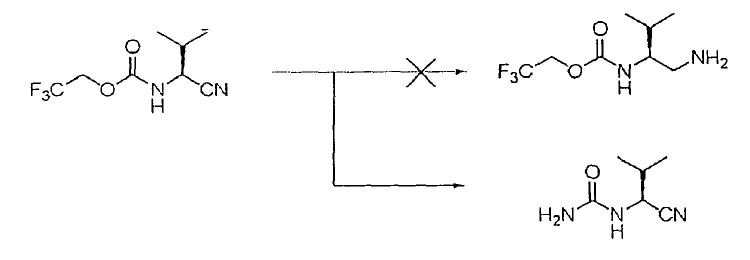

The reduction of the compound (II) was carried out with reference to Non-patent Document 2. In an autoclave, 30 ml of saturated ammonia ethanol containing 0.6 g of the compound (II) and 0.6 g of Raney nickel (a product of Wako Pure Chemical Industries, Ltd.) was pressurized with hydrogen gas to 0.35 MPa, and then the reaction was carried out at room temperature. After the reaction was carried out for 5 hours, and then the catalyst was filtered and the solvent was distilled off under reduced pressure. When IPE was added and the precipitate was filtered, the obtained compound was not a free body of the intended compound (III), but N-(aminocarbonyl)-L-valinonitrile (hereinafter referred to as the impurity (I)). As described above, it was determined that the intended compound could not be obtained by the conventional method.
Quantity of impurity (I): 0.16 g (Yield: 42%)
¹H NMR (DMSO-d₆)
δ0.95(3H,d,J=6.83Hz),0.98(3H,d,J=6.83Hz),1.94(1H,m),4.43(1H,m) ,5.78(2H,s),6.75(1H,brs).

### Example 9

### Synthesis (2S)-3-Methyl-N¹-toluoyl-N²-(2,2,2-trifluoroethoxycarbonyl)-but ane-1,2-diamine (hereinafter referred to as the compound (IV)) (1)

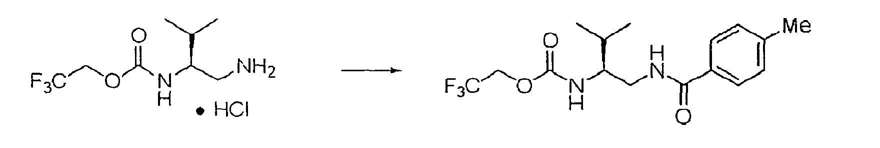

To 25 ml of water containing 1.91 g of sodium hydrogen carbonate were added 20 ml of ethyl acetate and 2.0 g of the compound (III), and the resulting mixture was stirred. 1.40 g of toluic acid chloride was added dropwise to the solution. The solution was stirred at room temperature for 2.5 hours, and then liquid separation was carried out. To the organic layer was added sodium sulfate and the organic layer was dried and filtered. Thereafter, the filtrate was concentrated under reduced pressure. 30 ml of IPE was further added to the redsidue, and the precipitate was fully washed and then filtered. The obtained white solid was the title compound.
Quantity: 2.33 g (Yield: 89%)

### Example 10

### Synthesis of Compound (IV) (2)

To a mixed solution of 20 ml of ethyl acetate and 30 ml of water was added 2.0 g of the compound (III), and then the pH was set to 8 by adding of a 8 weight % aqueous sodium hydroxide solution. Subsequently, an ethyl acetate solution containing 1.4 g of toluic acid chloride and a 8 weight % aqueous sodium hydroxide solution were added dropwise to the mixture while maintaining the pH at 7.5 to 8.5. After the completion of the reaction, liquid separation was carried out, and the organic layer was dried over sodium sulfate. Sodium sulfate was removed and then the solvent was distilled off under reduced pressure. Subsequently, IPE was added and the precipitate was filtered. The obtained white solid was the title compound.
Quantity: 2.19 g (Yield: 84%)

### Example 11

### Synthesis of (2S)-3-Methyl-N¹-(1-methyl-3-trifluoromethyl-1H-pyrazole-4-carb onyl)-N²-(2,2,2-trifluoroethoxycarbonyl)-butane-1,2-diamine

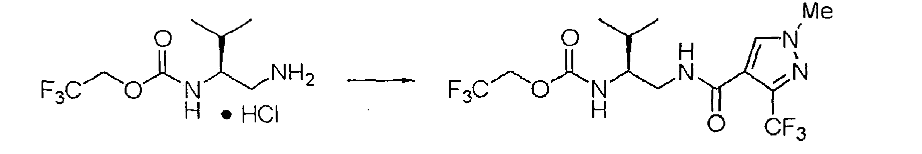

The reaction was carried out in the same manner as in Example 9, except that 1.0 g of the compound (III) was used, and 1-methyl-3-trifluoromethyl-1H-pyrazole-4-carbonyl chloride was used instead of toluic acid chloride.
Yield of white solid: 1.24 g (92%)
¹H NMR (CDCl₃)
δ0.98(3H,d,J=6.83Hz),1.00(3H,d,J=6.83Hz),1.85(1H,m),3.53(2H,m) ,3.66(1H,m),3.95(3H,s),4.42(2H,m),5.13(1H,brd),6.30(1H,brs),7. 86(1H,s).

### Example 12

### Synthesis of (2S)-3-methyl-N¹-(2,4-dichlorobenzoyl)-N²-(2,2,2-trifluoroethox ycarbonyl)-butane-1,2-diamine

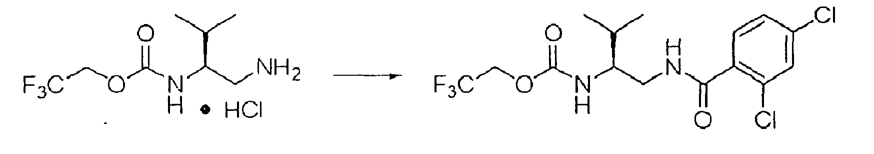

The reaction was carried out in the same manner as in Example 9, except that 0.5 g of the compound (III) was used, and 2,4-dichlorobenzoyl chloride was used instead of toluic acid chloride.
Yield of white solid: 0.64 g (96%)
¹H NMR (CDCl₃)
δ0.99(3H,d,J=6.83Hz),1.01(3H,d,J=6.83Hz),1.88(1H,m),3.53(1H,m) ,3.65(1H,m),3.68(1H,m),4.43(2H,m),5.16(1H,d,J=8.78Hz),6.50(1H, brs),7.30(1H,dd,J=1.95,8.29Hz),7.41(1H,d,J=1.95Hz),7.55(1H,d,J =8.23Hz).

### Example 13

### Synthesis of N-(2,2,2-trifluoroethoxycarbonyl)-L-leucinamide (hereinafter referred to as the compound (V))

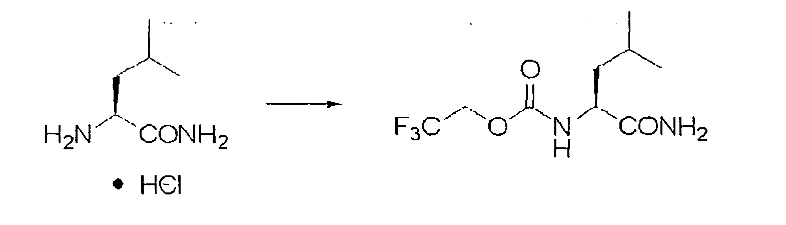

The reaction was carried out in the same manner as in Example 1, except that leucinamide hydrochloride was used instead of valinamide hydrochloride, and 5.0 g of leucinamide hydrochloride was used.
Quantity of white solid: 7.0 g (Yield: 91%)
¹H NMR(DMSO-d₆)
δ0.85(3H,d,J=6.34Hz),0.87(3H,d,J=6.83Hz),1.47(2H,m),1.59(1H,m) ,3.96(1H,m),4.69(2H,m),6.98(1H,s),7.36(1H,s),7.74(1H,d,J=8.29H z).

### Example 14

### Synthesis of N-(2,2,2-trifluoroethoxycarbonyl)-L-leucinonitrile (hereinafter referred to as the compound (VI))

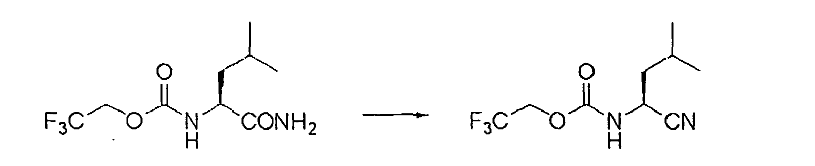

The reaction was carried out in the same manner as in Example 2, except that the compound (V) was used instead of the compound (I), and 5.0 g of the compound (V) was used. At that time, purification was carried out not by distillation, but by column chromatography.
Quantity of yellow oily substance: 4.41 g (95%)
¹H NMR(CDCl₃)
δ0.99(6H,d,J=6.34Hz),1.7-1.9(3H,m),4.47(1H,m),4.53(1H,m),4.62( 1H,m),5.31(1H,brd).

### Example 15

### Synthesis of (2S)-4-methyl-N²-(2,2,2-trifluoroethoxycarbonyl)-pentane-1,2-di amine hydrochloride (hereinafter referred to as the compound (VII))

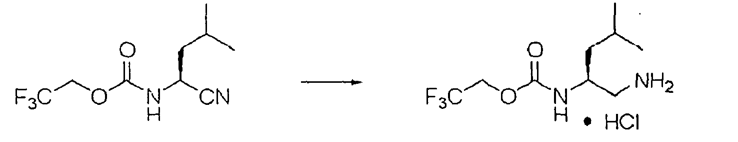

The reaction was carried out in the same manner as in Example 5, except that the compound (VI) was used instead of the compound (II), and 2.5 g of the compound (VI) was used.
Quantity of white solid: 2.32 g (Yield: 79%)
¹H NMR(DMSO-d₆)
δ0.86(3H,d,J=6.34Hz),0.88(3H,d,J=6.34Hz),1.27(1H,m),1.36(1H,m) ,1.56(1H,m),2.73(1H,dd,J=8.78,12.69Hz),2.82(1H,dd,J=4.39,12.69 Hz),3.76(1H,m),4.57(1H,m),4.69(1H,m),7.67(1H,d,J=8.78Hz),8.06(
3H,brs).

### Example 16

### Synthesis of (2S)-4-methyl-N¹-toluoyl-N²-(2,2,2-trifluoroethoxycarbonyl)-pen tane-1,2-diamine

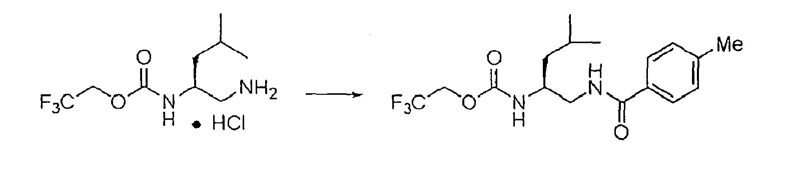

The reaction was carried out in the same manner as in Example 9, except that the compound (VII) was used instead of the compound (III), and 0.5 g of the compound (VII) was used.
Quantity of white solid: 0.56 g (Yield: 87%)
¹H NMR(CDCl₃)
δ0.94(3H,d,J=6.34Hz),0.95(3H,d,J=6.34Hz),1.38(1H,m),1.43(1H,m) ,1.70(1H,m),2.39(3H,s),3.49(1H,m),3.53(1H,m),3.92(1H,m),4.41(2 H,m),5.19(1H,d,J=8.78Hz),6.71(1H,brs),7.22(2H,d,J=7.81Hz),7.65 (2H,d,J=7.81Hz).

### Example 17

### Synthesis of (2S)-4-methyl-N¹-(1-methyl-3-trifluoromethyl-1H-pyrazole-4-carb onyl)-N²-(2,2,2-trifluoroethoxycarbonyl)-pentane-1,2-diamine

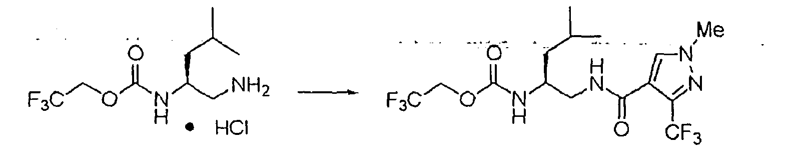

The reaction was carried out in the same manner as in Example 9, except that the compound (VII) was used instead of the compound (III), 1-methyl-3-trifluoromethyl-1H-pyrazole-4-carbonyl chloride was used instead of toluic acid chloride, and 0.3 g of the compound (VII) was used.
Yield of white solid: 0.36 g (Yield: 80%)
¹H NMR(CDCl₃)
δ0.93(3H,d,J=6.34Hz),0.95(3H,d,J=6.34Hz),1.38(2H,m),1.68(1H,m) ,3.45(1H,m),3.54(1H,m),3.89(1H,m),3.96(3H,s),4.43(2H,m),5.05(1 H,d,J=8.78Hz),6.34(1H,brs),7.87(1H,s).

### Example 18

### Synthesis of (2S)-4-Methyl-N¹-(2,4-dichlorobenzoyl)-N²-(2,2,2-trifluoroethox ycarbonyl)-pentane-1,2-diamine

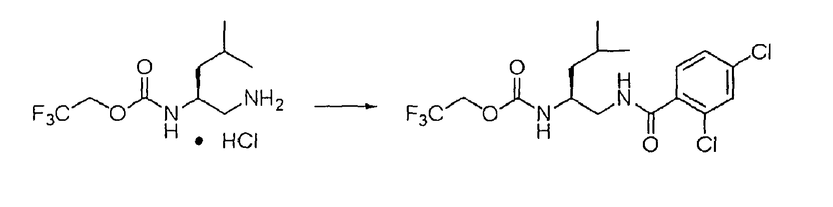

The reaction was carried out in the same manner as in Example 9, except that the compound (VII) was used instead of the compound (III), 2,4-dichlorobenzoyl chloride was used instead of toluic acid chloride, and 0.2 g of the compound (VII) was used.
Quantity of white solid: 0.29 g (Yield: 97%)
¹H NMR(CDCl₃)
δ0.94(3H,d,J=5.37Hz),0.96(3H,d,J=6.34Hz),1.42(2H,m),1.71(1H,m) ,3.54(2H,m),3.93(1H,m),4.42(2H,m),5.08(1H,d,J=8.30Hz),6.57(1H, brs),7.30(1H,dd,J=1.95,8.29Hz),7.41(1H,d,J=1.95Hz),7.56(1H,d,J =8.29Hz).

### Example 19

### Synthesis of N-(2,2,2-trifluoroethoxycarbonyl)-L-isoleucinamide (hereinafter referred to as the compound (VIII))

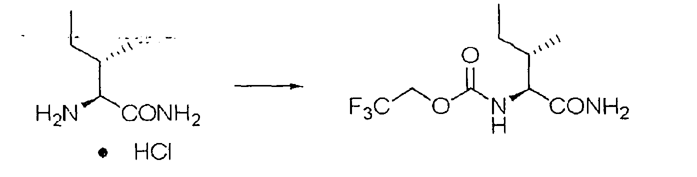

The reaction was carried out in the same manner as in Example 1, except that isoleucinamide hydrochloride was used instead of valinamide hydrochloride, and 5.0 g of isoleucinamide hydrochloride was used.
Quantity of white solid: 7.31 g (Yield: 95%)
¹H NMR(DMSO-d₆)
δ0.82(6H,m),1.13(1H,m),1.41(1H,m),1.71(1H,m),3.81(1H,t,J=8.29H z),4.64(2H,
q,J=9.27Hz),7.05(1H,s),7.39(1H,s),7.65(1H,d,J=8.29Hz).

### Example 20

### Synthesis of N-(2,2,2-trifluoroethoxycarbonyl)-L-isoleucinonitrile (hereinafter referred to as the compound (IX))

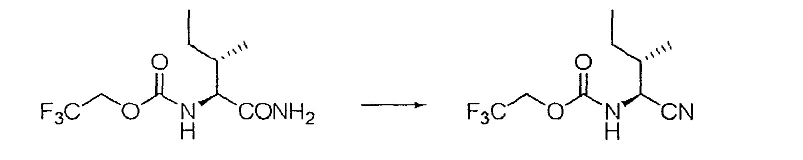

The reaction was carried out in the same manner as in Example 2, except that the compound (VIII) was used instead of the compound (I), and 5.0 g of the compound (VIII) was used. At that time, purification was carried out not by distillation, but by column chromatography.
Quantity of colorless oily substance: 4.53 g (Yield: 97%)
¹H NMR(CDCl₃)
δ0.98(3H,t,J=7.32Hz),1.10(3H,d,J=6.83Hz),1.34(1H,m),1.59(1H,m) ,1.83(1H,m),4.48(1H,m),4.53(1H,m),4.59(1H,m),5.35(1H,brd).

### Example 21

### Synthesis of (2S,3S)-3-methyl-N²-(2,2,2-trifluoroethoxycarbonyl)-pentane-1,2 -diamine hydrochloride (hereinafter referred to as the compound (X))

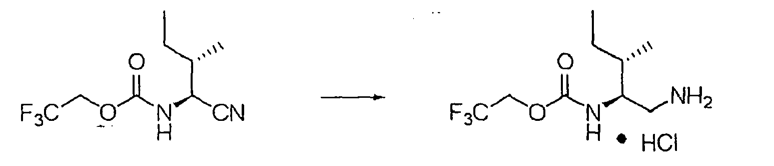

The reaction was carried out in the same manner as in Example 5, except that the compound (IX) was used instead of the compound (II), and 2.5 g of the compound (IX) was used.
Quantity of light peach solid: 2.56 g (Yield: 92%)
¹H NMR(DMSO-d₆)
δ0.84(6H,m),1.11(1H,m),1.36(1H,m),1.53(1H,m),2.75(1H,dd,J=10.2 5,12.69Hz),2.92(1H,dd,J=2.93,12.69Hz),3.60(1H,m),4.55(1H,m),4. 72(1H,m),7.73(1H,d,J=8.78Hz),8.10(3H,brs).

### Example 22

### Synthesis of (2S,3S)-3-methyl-N¹-toluoyl-N²-(2,2,2-trifluoroethoxycarbonyl)-pentane-1,2-diamine

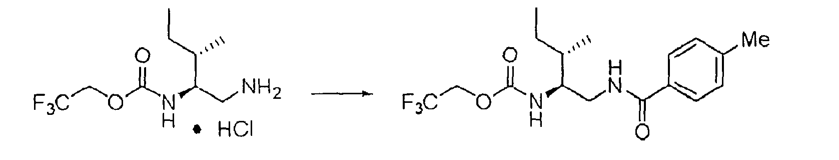

The reaction was carried out in the same manner as in Example 9, except that the compound (X) was used instead of the compound (III), and 0.5 g of the compound (X) was used.
Quantity of white solid: 0.56 g (Yield: 87%)

### Example 23

### Synthesis of (2S,3S)-3-methyl-N¹-(1-methyl-3-trifluoromethyl-1H-pyrazole-4-c arbonyl)-N²-(2,2,2-trifluoroethoxycarbonyl)-pentane-1,2-diamine

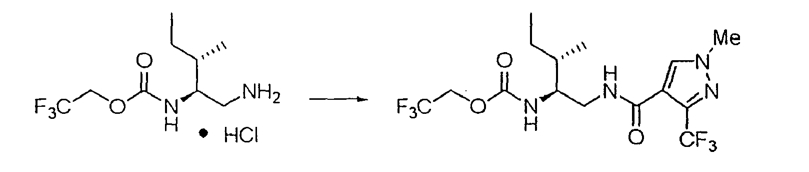

The reaction was carried out in the same manner as in Example 9, except that the compound (X) was used instead of the compound (III), 1-methyl-3-trifluoromethyl-1H-pyrazole-4-carbonyl chloride was used instead of toluic acid chloride, and 0.3 g of the compound (X) was used.
Quantity of white solid: 0.45 g (Yield: > 99%)
¹H NMR(CDCl₃)
δ0.94(3H,t,J=7.32Hz),0.97(3H,d,J=6.83Hz),1.19(1H,m),1.5-1.6(2H ,m),3.53(2H,m),3.72(1H,m),3.95(3H,s),4.43(2H,m),5.16(1H,brd),6 .30(1H,brs),7.85(1H,s).

### Example 24

### Synthesis of (2S,3S)-3-methyl-N¹-(2,4-dichlorobenzoyl)-N²-(2,2,2-trifluoroet hoxycarbonyl)-pentane-1,2-diamine

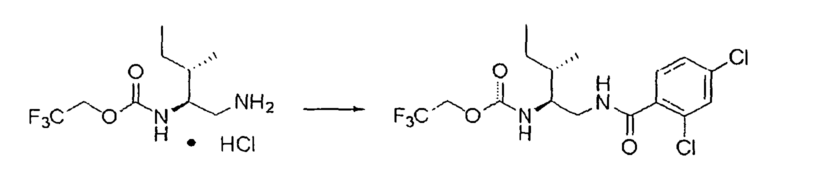

The reaction was carried out in the same manner as in Example 9, except that the compound (X) was used instead of the compound (III), 2,4-dichlorobenzoyl chloride was used instead of toluic acid chloride, and 0.2 g of the compound (X) and 2,4-dichlorobenzoyl chloride was used.
Quantity of white solid: 0.27 g (Yield: 90%)
¹H NMR(CDCl₃)
δ0.95(3H,t,J=7.32Hz),0.99(3H,d,J=6.83Hz),1.22(1H,m),1.55(1H,m) ,1.67(1H,m),3.52(1H,m),3.64(1H,m),3.76(1H,m),4.42(2H,m),5.21(1 H,d,J=8.78Hz),6.50(1H,brs),7.29(1H,dd,J=1.95,8.29Hz),7.41(1H,d ,J=1.95Hz),7.54(1H,d,J=8.29Hz).

### Example 25

### Synthesis of N-(2,2,2-trifluoroethoxycarbonyl)-L-phenylalaninamide (hereinafter referred to as the compound (XI))

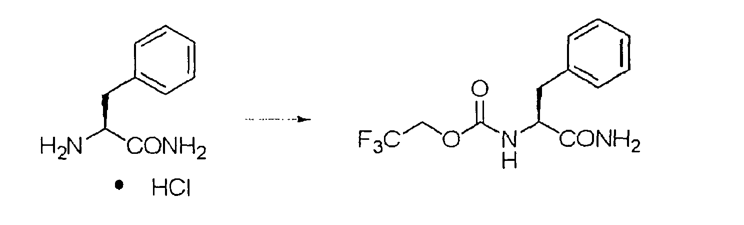

The reaction was carried out in the same manner as in Example 1, except that phenylalaninamide hydrochloride was used instead of valinamide hydrochloride, and 5.0 g of phenylalaninamide hydrochloride was used.
White solid: 6.67 g Quantity: 6.76 g (Yield: 93%)
¹H NMR(DMSO-d₆)
δ2.75(1H,m),2.99(1H,m),4.15(1H,m),4.57(2H,m),7.09(1H,brs),7.20 (1H,m),7.24(1H,m),7.27(3H,m),7.51(1H,brs),7.85(1H,d,J=8.19Hz).

### Example 26

### Synthesis of N-(2,2,2-trifluoroethoxycarbonyl)-L-phenylalaninonitrile (hereinafter referred to as the compound (XII))

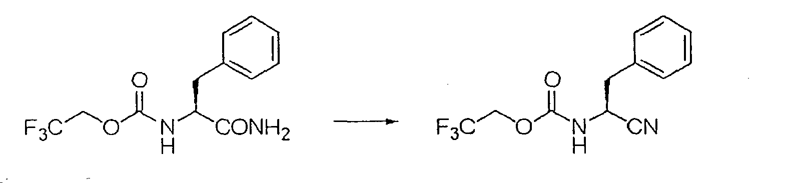

The reaction was carried out in the same manner as in Example 2, except that the compound (XI) was used instead of the compound (I), and 5.0 g of the compound (XI) was used. At that time, purification was carried out not by distillation, but by column chromatography.
White solid: 3.97 g (Yield: 85%)
¹H NMR(CDCl₃)
δ3.13(2H,m),4.49(2H,m),4.86(1H,m),5.29(1H,brd),7.28(2H,m),7.37 (3H,m).

### Example 27

### Synthesis of (2S)-N²-(2,2,2-trifluoroethoxycarbonyl)-3-phenyl-propane-1,2-di amine hydrochloride (hereinafter referred to as the compound (XIII))

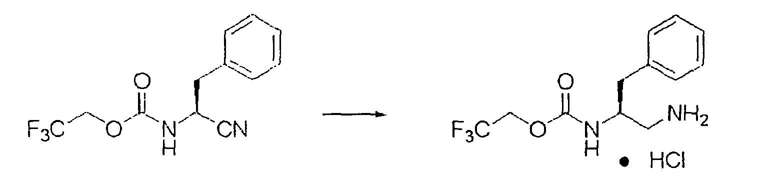

The reaction was carried out in the same manner as in Example 5, except that the compound (XII) was used instead of the compound (II), and 2.0 g of the compound (XII) was used.
Quantity of white solid: 2.04 g (Yield: 88%)
¹H NMR(DMSO-d₆)
δ2.72(1H,m),2.85(3H,m),3.91(1H,m),4.56(2H,m),7.21(3H,m),7.30(2 H,m),7.80(1H,d,J=8.78Hz),8.09(3H,brs).

### Example 28

### Synthesis of (2S)-N¹-toluoyl-N²-(2,2,2-trifluoroethoxycarbonyl)-3-phenyl-pro pane-1,2-diamine

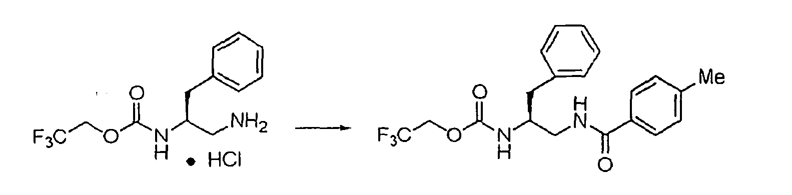

The reaction was carried out in the same manner as in Example 9, except that the compound (XIII) was used instead of the compound (III), and 0.3 g of the compound (XIII) was used.
White solid: 0.35 g (Yield: 93%)
¹H NMR(CDCl₃)
δ2.39(3H,s),2.83(1H,dd,J=7.81,14.15Hz),3.00(1H,dd,J=6.83,14.15 Hz),3.53(1H,m),3.59(1H,m),4.11(1H,m),4.40(2H,m),5.63(1H,d,J=7. 81Hz),6.52(1H,brs),7.24(5H,m),7.33(2H,m),7.62(2H,d,J=7.81Hz).

### Example 29

### Synthesis of (2S)-N¹-(1-methyl-3-trifluoromethyl-1H-pyrazole-4-carbonyl)-N²-(2,2,2-trifluoro-ethoxycarbonyl)-3-phenyl-propane-1,2-diamine

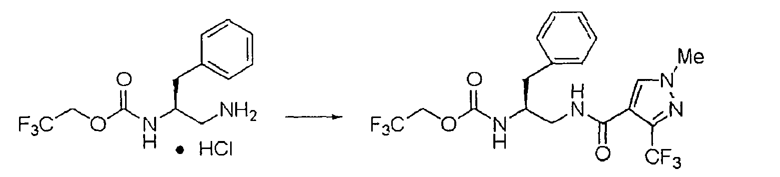

The reaction was carried out in the same manner as in Example 9, except that the compound (XIII) was used instead of the compound (III), 1-methyl-3-trifluoromethyl-1H-pyrazole-4-carbonyl chloride was used instead of toluic acid chloride, and 0.2 g of the compound (XIII) was used.
Quantity of white solid: 0.28 g (Yield: 97%)
¹H NMR(CDCl₃)
δ2.81(1H,dd,J=7.81,14.15Hz),2.97(1H,dd,J=6.83,14.15Hz),3.52(2H ,m),3.96(3H,s),4.03(1H,m),4.41(2H,m),5.50(1H,d,J=7.81Hz),6.30( 1H,brs),7.20(2H,m),7.24(1H,m),7.33(2H,m),7.88(1H,s).

### Example 30

### Synthesis of N-(2,2,2-trifluoroethoxycarbonyl)-L-alaninamide (hereinafter referred to as the compound (XIV))

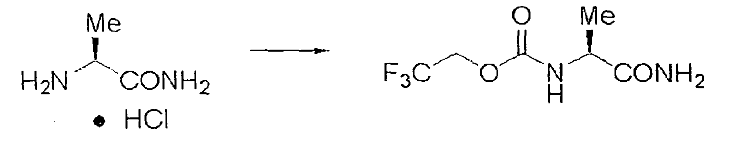

15.0 g of alaninamide hydrochloride was added to 250 ml of water containing 25.29 g of sodium hydrogen carbonate and 300 ml of ethyl acetate, and the resulting mixture was stirred until it became uniform. An ethyl acetate solution containing 23.48 g of 2,2,2-trifluoroethoxycarbonyl chloride was added dropwise to the solution at room temperature over 30 minutes. The solution was stirred at the same temperature for 2 hours, and then liquid separation was carried out. The organic layer was dried over sodium sulfate and filtered, and then the solvent was distilled off under reduced pressure. To the residue was added hexane and the solution was stirred. Thereafter, the precipitate was filtered to obtain the title compound.
Quantity of white solid: 24.61 g (Yield: 95%)
¹H NMR(DMSO-d₆)
δ1.21(3H,d,J=7.32Hz),3.96(1H,m),4.62(2H,m),6.98(1H,brs),7.33(1 H,brs),7.76(1H,d,J=7.81Hz).

### Example 31

### Synthesis of N-(2,2,2-trifluoroethoxycarbonyl)-L-alaninonitrile (hereinafter referred to as the compound (XV))

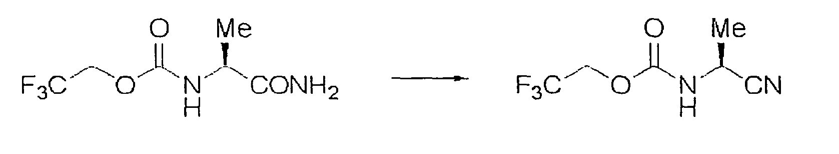

The reaction was carried out in the same manner as in Example 2, except that the compound (XIV) was used instead of the compound (I), and 31.0 g of the compound (XIV) was used. At that time, purification was carried out not by distillation, but by column chromatography.
Quantity of white solid: 25.83 g (Yield: 91%)
¹H NMR(CDCl₃)
δ1.61(3H,d,J=7.32Hz),4.47(1H,m),4.53(1H,m),4.67(1H,m),5.38(1H, brd).

### Example 32

### Synthesis of (2S)-N²-(2,2,2-trifluoroethoxycarbonyl)-propane-1,2-diamine hydrochloride (hereinafter referred to as the compound (XVI))

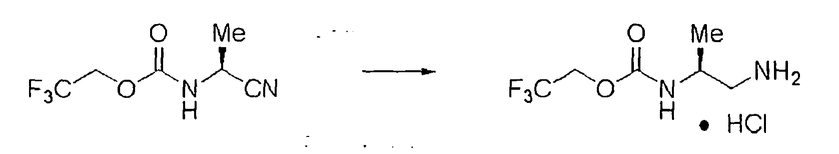

The reaction was carried out in the same manner as in Example 5, except that the compound (XV) was used instead of the compound (II), and 2.0 g of the compound (XV) was used.
Quantity of white solid: 2.05 g (Yield: 85%)
¹H NMR(DMSO-d₆)
δ1.12(3H,t,J=6.83Hz),2.81(2H,m),3.79(1H,m),4.60(1H,m),4.67(1H, m),7.76(1H,d,J=8.29Hz),8.12(3H,brs).

### Example 33

### Synthesis of (2S)-N¹-toluoyl-N²-(2,2,2-trifluoroethoxycarbonyl)-propane-1,2-diamine

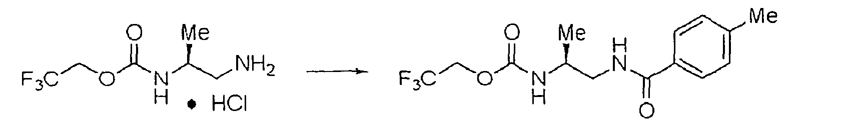

The reaction was carried out in the same manner as in Example 9, except that the compound (XVI) was used instead of the compound (III), and 0.5 g of the compound (XVI) was used.
Quantity of white solid: 0.56 g (Yield: 84%)
¹H NMR(CDCl₃)
δ1.26(3H,d,J=6.83Hz),2.39(3H,s),3.53(2H,m),3.95(1H,m),4.41(2H, m),5.50(1H,brd,J=7.32Hz),6.74(1H,brs),7.22(2H,d,J=7.81Hz),7.66 (2H,d,J=7.81Hz)

### Example 34

### Synthesis of (2S)N¹-(1-methyl-3-trifluoromethyl-1H-pyrazole-4-carbonyl)-N²-( 2,2,2-trifluoroethoxycarbonyl)-propane-1,2-diamine

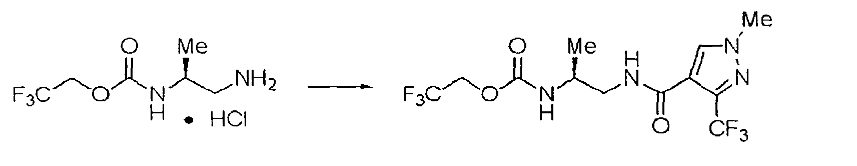

The reaction was carried out in the same manner as in Example 9, except that the compound (XVI) was used instead of the compound (III), 1-methyl-3-trifluoromethyl-1H-pyrazole-4-carbonyl chloride was used instead of toluic acid chloride, and 0.3 g of the compound (XVI) was used.
Quantity of orange solid: 0.38 g (Yield: 79%)
¹H NMR(CDCl₃)
δ1.24(3H,d,J=6.83Hz),3.50(2H,m),3.93(1H,m),3.96(3H,s),4.42(2H, m),5.34(1H,d,J=7.32Hz),6.39(1H,brs),7.89(1H,s).

### Example 35

### Synthesis of N-(2,2,2-trifluoroethoxycarbonyl)-L-prolinamide (hereinafter referred to as the compound (XV-2))

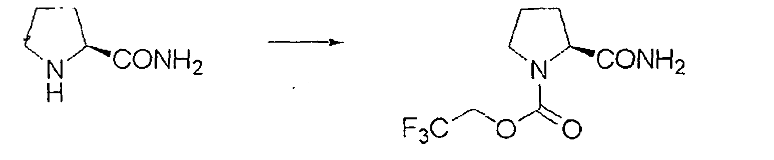

To 65 ml of water containing 5.0 g of prolinamide was added dropwise 7 ml of dioxane containing 8.54 g of 2,2,2-trifluoroethoxycarbonyl chloride at room temperature. When the pH of the reaction solution became 8, dropwise addition of a 8 weight % aqueous sodium hydroxide solution also started at the same time, and the pH of the reaction solution was maintained at 8 ± 0.5. After the completion of dropwise addition, the solution was stirred for 2 hours, and then ethyl acetate was added to the solution to carry out liquid separation. The organic layer was dried over sodium sulfate and filtered, and then the filtrate was put under reduced pressure to remove the solvent. The obtained solid was the title compound.
Quantity of white solid: 8.48 g (Yield: 81%)
¹H NMR(DMSO-d₆)
δ1.82(3H,m),2.16(1H,m),3.38(1H,m),3.46(1H,m),4.13(1H,m),4.6-4. 7(2H,m),6.99(1H,s),7.41(1H,s).

### Example 36

### Synthesis of N-(2,2,2-trifluoroethoxycarbonyl)-L-prolinonitrile (hereinafter referred to as the compound (XVI-2))

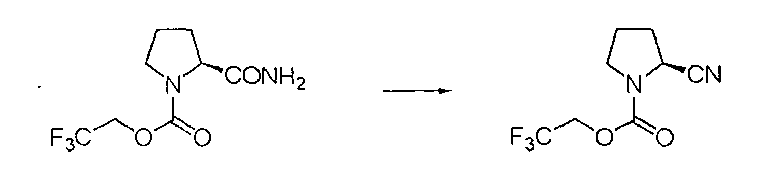

The reaction was carried out in the same manner as in Example 2, except that the compound (XV-2) was used instead of the compound (I), and 5.0 g of the compound (XV-2) was used. At that time, purification was carried out not by distillation, but by column chromatography.
Quantity of yellow transparent oily substance: 4.13 g (Yield: 89%)
¹H NMR(CDCl₃)
δ2.1-2.3(4H,m),3.46(1H,m),3.63(1H,m),4.49(1H,m),4.61(2H,m).

### Example 37

### Synthesis of (2S)-N-(2,2,2-trifluoroethoxycarbonyl)-2-(aminomethyl)-pyrroli dine hydrochloride (hereinafter referred to as the compound (XVII))

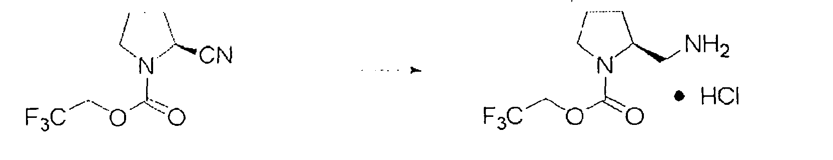

The reaction was carried out in the same manner as in Example 5, except that the compound (XVI-2) was used instead of the compound (2), and 2.0 g of the compound (XVI-2) was used.
Quantity of white solid: 1.67 g (Yield: 71%)
¹H NMR(DMSO-d₆)
δ1.8-2.0(4H,m),2.86(1H,m),2.96(1H,m),3.38(2H,m),4.03(1H,m),4.6 9(2H,m),8.19(3H,brs).

### Example 38

### Synthesis of (2S)-N-(2,2,2-trifluoroethoxycarbonyl)-2-(N-toluoyl-aminomethy 1)-pyrrolidine

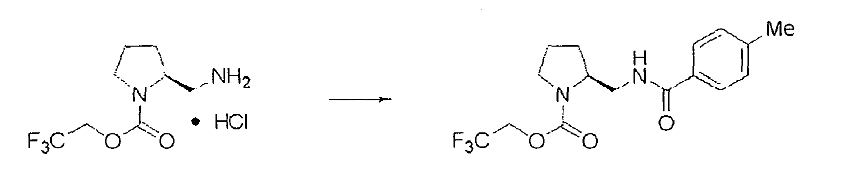

The reaction was carried out in the same manner as in Example 9, except that the compound (XVII) was used instead of the compound (III), and 0.5 g of the compound (XVII) was used.
Quantity of white solid: 0.50 g (Yield: 80%)
¹H NMR(CDCl₃)
δ1.8-2.2(4H,m),2.39(3H,s),3.4-3.5(3H,m),3.68(1H,m),4.19(1H,m), 4.52(2H,m),7.23(2H,d,J=8.29Hz),7.72(2H,d,J=8.29Hz),7.79(1H,brs ).

### Example 39

### Synthesis of (2S)-N-(2,2,2-trifluoroethoxycarbonyl)-2{N-(1-methyl-3-trifluo romethyl-1H-pyrazole-4-carbonyl)-aminomethyl}-pyrrolidine

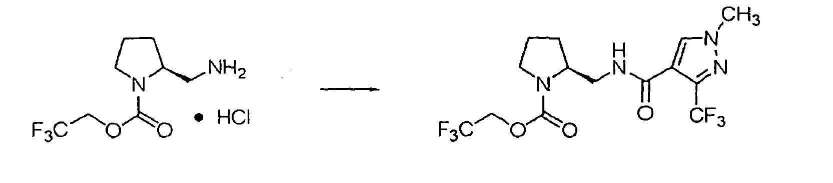

The reaction was carried out in the same manner as in Example 9, except that the compound (XVII) was used instead of the compound (III), 1-methyl-3-trifluoromethyl-1H-pyrazole-4-carbonyl chloride was used instead of toluic acid chloride, and 0.3 g of the compound (XVII) was used.
Quantity of colorless and transparent oil: 0.46 g (Yield: > 0.99%)
¹H NMR(CDCl₃)
δ1.8-2.2(4H,m),3.4-3.7(4H,m),3.96(3H,s),4.10(1H,m),4.49(2H,m), 7.11(1H,brs),7.80(1H,s).

### Example 40

### Synthesis of (2S)-N-(2,2,2-trifluoroethoxycarbonyl)-2{N-(2,4-dichlorobenzoy 1)-aminomethyl}-pyrrolidine

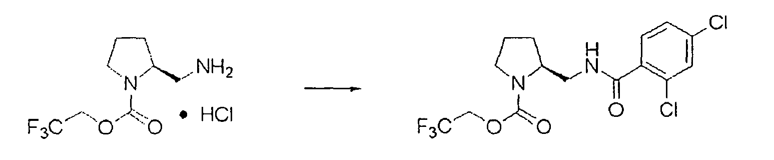

The reaction was carried out in the same manner as in Example 9, except that the compound (XVII) was used instead of the compound (III), 2,4-dichlorobenzoyl chloride was used instead of toluic acid chloride, and 0.3 g of the compound (XVII) was used.
Quantity of white solid: 0.37 g (Yield: 81%)
¹H NMR(CDCl₃)
δ1.7-2.2(4H,m),3.4-3.8(4H,m),9.13(1H,m),4.48(2H,m),7.29(1H,dd, J=1.95,8.30Hz),7.34(1H,brd),7.42(1H,d,J=1.95Hz),7.53(1H,d,J=8. 30Hz).

### Example 41

### Synthesis of N-methyl-N-(2,2,2-trifluoroethoxycarbonyl)-L-valinamide (hereinafter referred to as the compound (XVIII))

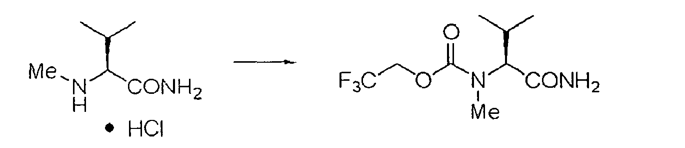

To 60 ml of water containing 3.0 g of N-methyl-L-valinamide and 4.54 g of sodium hydrogen carbonate was added dropwise 6 ml of dioxane containing 3.51 g of 2,2,2-trifluoroethoxycarbonyl chloride at room temperature over 20 minutes. The resulting solution was stirred for 2 hours, and then ethyl acetate was added to the solution , and carried out liquid separation. The obtained organic layer was dried over sodium sulfate and filtered, and then the solvent was distilled off under reduced pressure. The obtained compound was the title compound.
Quantity of colorless oily substance: 4.61 g (Yield: > 99%)
¹H NMR(CDCl₃)
δ0.89(3H,d),0.99(3H,d),2.28(1H,m),2.94(3H,s,major),2.95(3H,s,m inor),4.09(1H,d),4.51(2H,m),5.60(1H,brs),6.04(1H,brs).

### Example 42

### Synthesis of N-methyl-N-(2,2,2-trifluoroethoxycarbonyl)-L-valinonitrile (hereinafter referred to as the compound (XIX))

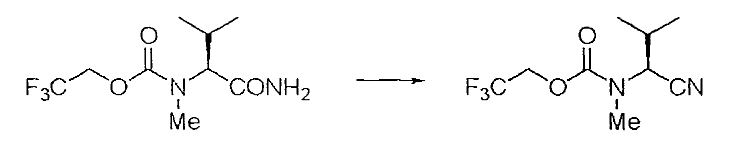

The reaction was carried out in the same manner as in Example 2, except that the compound (XVIII) was used instead of the compound (I), and 4.27 g of the compound (XVIII) was used. At that time, purification was carried out not by distillation, but by column chromatography.
Quantity of colorless oily substance: 3.36 g (Yield: 85%)
¹H NMR(CDCl₃)
δ0.94(3H,d,J=6.34Hz),1.18(3H,d,J=6.34Hz),2.13(1H,m),3.01(3H,s) ,4.4-4.6(2H,m),4.80(1H,d,J=10.73Hz).

### Example 43

### Synthesis of (2S)-3-methyl-N²-methyl-N²-(2,2,2-trifluoroethoxycarbonyl)-buta ne-1,2-diamine hydrochloride (hereinafter referred to as the compound (XX))

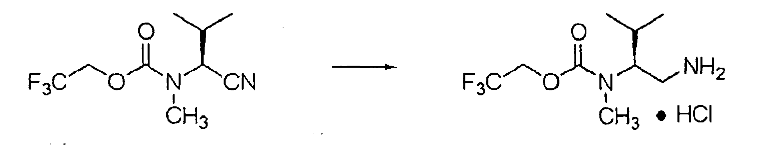

The reaction was carried out in the same manner as in Example 5, except that the compound (XIX) was used instead of the compound (II), and 2.40 g of the compound (XIX) was used.
Quantity of white solid: 2.49 g (Yield: 89%)
¹H NMR(DMSO-d₆)δ0.77(3H,d,J=6.34Hz)0.93(3H,d,J=6.34Hz)1.83(1H,m) ,2.76(3H,s),3.00(2H,m),3.77(1H,m),4.60(1H,m),4.77(1H,m),7.99(3 H,brs).

### Example 44

### Synthesis of (2S)-3-methyl-N²-methyl-N¹-{4-(trifluoromethyl)benzoyl}-N²-(2,2 ,2-trifluoroethoxycarbonyl)-butane-1,2-diamine

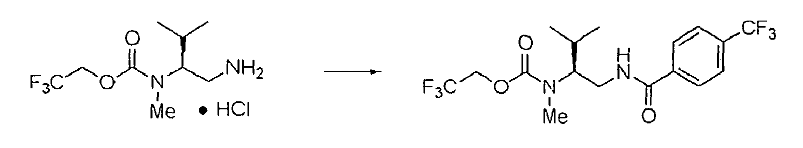

The reaction was carried out in the same manner as in Example 9, except that the compound (XX) was used instead of the compound (III), 4-(trifluoromethyl)benzoyl chloride was used instead of toluic acid chloride, and 0.3 g of the compound (XX) was used.
Quantity of white solid: 0.43 g (Yield: 96%)
¹H NMR(CDCl₃)
δ0.93(3H,m),1.07(3H,m),1.95(1H,m),2.85(3H,s,major),2.86(3H,s,m inor),3.55(1H,m),3.78-3.89(2H,m),4.45(1H,m),4.53(1H,m),6.15(1H ,brs,J=minor),6.55(1H,brs,major),7.78(2H,m),7.81(2H,m).

### Example 45

### Synthesis of (2S)-3-methyl-N¹-{4-(trifluoromethyl)benzoyl)-N²-(benzofuran-2-carbonyl)-butane-1,2-diamine

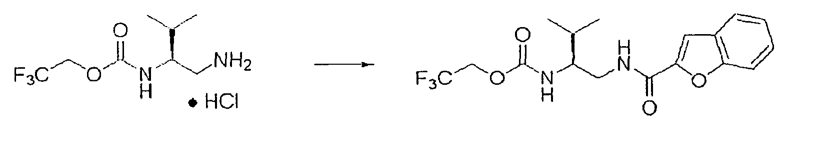

To 5 ml of THF containing 0.28 g of benzofuran-2-carboxylic acid was added 0.33 g of N,N'-carbonyldiimidazole and the resulting mixture was stirred for 1 hour to prepare N-(benzofuran-2-carbonyl)-imidazole. 0.23 g of imidazole and 0.3 g of the compound (III) were sequentially added to the solution and reacted for 3 hours. Ethyl acetate and water were added to the solution , and to carried out liquid separation. Thereafter, 1N hydrochloride, a saturated aqueous solution of sodium hydrogen carbonate and a saturated sodium chloride solution were sequentially separated from the organic layer. The obtained organic layer was dried over sodium sulfate and filtered, and then the solvent was distilled off under reduced pressure. To the residue was added IPE and the white precipitate was filtered. The obtained compound was the title compound.
Quantity of white solid: 0.35 g (Yield: 84%)

According to the present invention, it is possible to provide a process which is advantageous to industrial production of ethylenediamine derivatives having a halogenated carbamate group and an acyl group, and its value for industrial application is high.

## Claims

1. A process for producing a compound represented by the general formula (4) comprising;
converting the compound represented by the general formula (1) into a compound represented by.the general formula (2) by performing the catalytic hydrogenation of a compound represented by the general formula (1) in the presence of an acid, and
reacting the compound represented by the general formula (2) with a compound represented by the general formula (3), 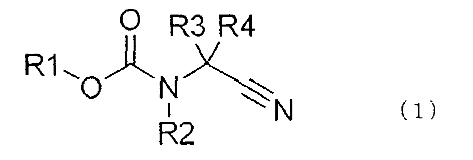 wherein, in the formula, R1 represents an alkyl group having 1 to 6 carbon atoms which is substituted with at least one halogen atom or a cycloalkyl group having 3 to 6 carbon atoms which is substituted with at least one halogen atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom), 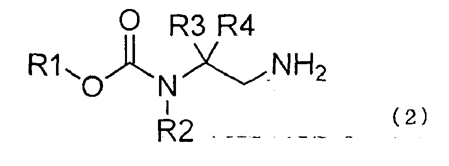 wherein, in the formula, R1, R2, R3 and R4 are the same as those described above, 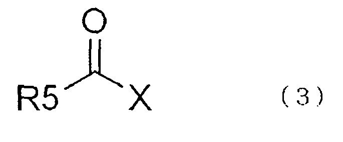 wherein, in the formula, R5 represents an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and X represents a leaving group, 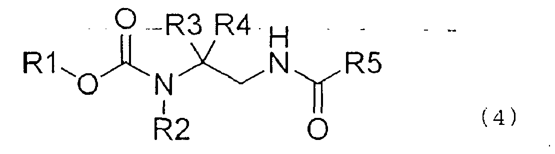 wherein, in the formula, R1, R2, R3, R4 and R5 are the same as those described above.

2. The process for producing a compound represented by the general formula (4) as set forth in claim 1, in which the compound represented by the general formula (1) is obtained by reacting a compound represented by the general formula (5) with a deoxidizing agent, 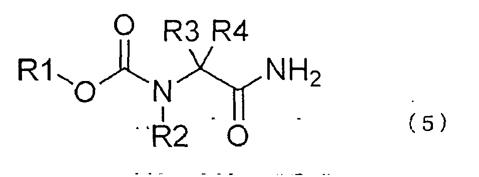 wherein, in the formula, R1, R2, R3 and R4 are the same as those described above.

3. The process for producing a compound represented by the general formula (4) as set forth in claim 2, in which the compound represented by the general formula (5) is obtained by reacting a compound represented by the general formula (6) with a compound represented by the general formula (7) in the presence of water, 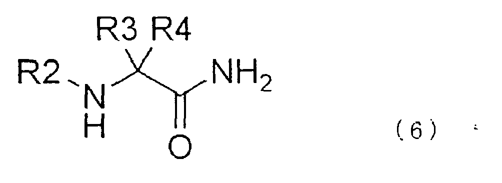 wherein, in the formula, R2, R3 and R4 are the same as those described above, 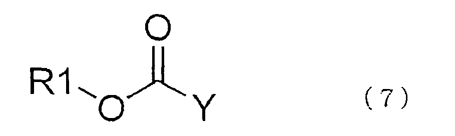 wherein, in the formula, R1 is the same as those described above; and Y represents a halogen atom.

4. The process for producing a compound represented by the general formula (4) as set forth in claim 1, in which, in the formulas, R1 represents an alkyl group having 1 to 6 carbon atoms which is substituted with at least one halogen atom; R2 represents a hydrogen atom or an alkyl group having 1 to 6 carbon atoms; R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom); and R5 represents an aryl group which may be substituted or a heteroaryl group which may be substituted.

5. The process for producing a compound represented by the general formula (4) as set forth in claim 2, in which, in the formulas, R1 represents an alkyl group having 1 to 6 carbon atoms which is substituted with at least one halogen atom; R2 represents a hydrogen atom or an alkyl group having 1 to 6 carbon atoms; R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom); and R5 represents an aryl group which may be substituted or a heteroaryl group which may be substituted.

6. The process for producing a compound represented by the general formula (4) as set forth in claim 3, in which, in the formula, R1 represents an alkyl group having 1 to 6 carbon atoms which is substituted with at least one halogen atom; R2 represents a hydrogen atom or an alkyl group having 1 to 6 carbon atoms; R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom); and R5 represents an aryl group which may be substituted or a heteroaryl group which may be substituted.

7. The process for producing a compound represented by the general formula (4) as set forth in claim 4, in which, in the formula, R1 is an alkyl group having 1 to 6 carbon atoms which is substituted with at least one fluorine atom.

8. The process for producing a compound represented by the general formula (4) as set forth in claim 5, in which, in the formula, R1 is an alkyl group having 1 to 6 carbon atoms which is substituted with at least one fluorine atom.

9. The process for producing a compound represented by the general formula (4) as set forth in claim 6, in which, in the formula, R1 is an alkyl group having 1 to 6 carbon atoms which is substituted with at least one fluorine atom.

10. A process for producing a compound represented by the general formula (2) comprising;
converting the compound represented by the general formula (1) into a compound represented by the general formula (2) by performing the catalytic hydrogenation of a compound represented by the general formula (1) in the presence of an acid, 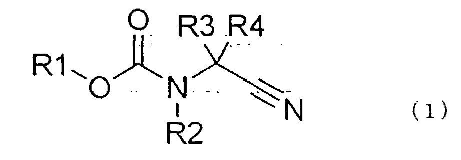
wherein, in the formula, R1 represents an alkyl group having 1 to 6 carbon atoms which is substituted with at least one halogen atom or a cycloalkyl group having 3 to 6 carbon atoms which is substituted with at least one halogen atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom), 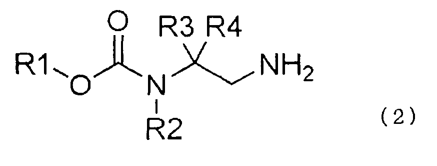
wherein, in the formula, R1, R2, R3 and R4 are the same as those described above.

11. The process for producing a compound represented by the general formula (2) as set forth in claim 10, in which, in the formulas, R1 represents an alkyl group having 1 to 6 carbon atoms which is substituted with at least one halogen atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom).

12. The process for producing a compound represented by the general formula (2) as set forth in claim 11, in which, in the formula, R1 is an alkyl group having 1 to 6 carbon atoms which is substituted with at least one fluorine atom.

13. A process for producing a compound represented by the general formula (1) comprising;
converting the resulting product into a compound represented by the general formula (1) by reacting a compound represented by the general formula (5) with a deoxidizing agent, 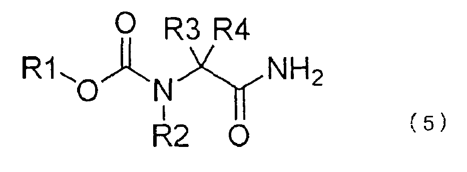 wherein, in the formula, R1 represents an alkyl group having 1 to 6 carbon atoms which is substituted with at least one halogen atom or a cycloalkyl group having 3 to 6 carbon atoms which is substituted with at least one halogen atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom), 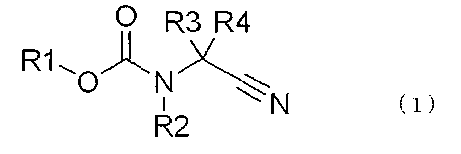 wherein, in the formula, R1, R2, R3 and R4 are the same as those described above.

14. The process for producing a compound represented by the general formula (1) as set forth in claim 13, in which, in the formulas, R1 represents an alkyl group having 1 to 6 carbon atoms which is substituted with at least one halogen atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom).

15. The process for producing a compound represented by the general formula (1) as set forth in claim 14, in which, in the-formula, R1 is an alkyl group having 1 to 6 carbon atoms which is substituted with at least one fluorine atom.

16. A process for producing a compound represented by the general formula (5) comprising;
converting the resulting product into a compound represented by the general formula (5) by reacting a compound represented by the general formula (6) with a compound represented by the general formula (7) in the presence of water, 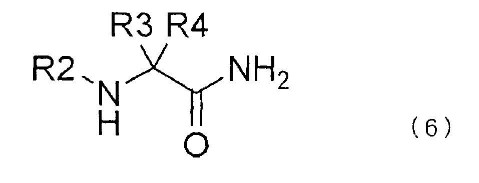 wherein, in the formula, R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom), 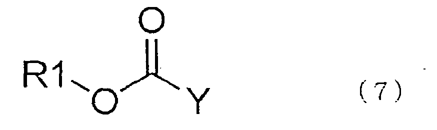 wherein, in the formula, R1 represents an alkyl group having 1 to 6 carbon atoms which is substituted with at least one halogen atom or a cycloalkyl group having 3 to 6 carbon atoms which is substituted with at least one halogen atom; and Y represents a halogen atom, 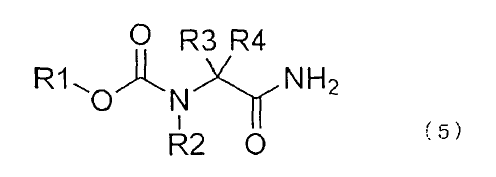 wherein, in the formula, R1, R2, R3 and R4 are the same as those described above.

17. The process for producing a compound represented by the general formula (5) as set forth in claim 16, in which, in the formulas, R1 represents an alkyl group having 1 to 6 carbon atoms which is substituted with at least one halogen atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom).

18. The process for producing a compound represented by the general formula (5) as set forth in claim 17, in which, in the formula, R1 is an alkyl group having 1 to 6 carbon atoms which is substituted with at least one fluorine atom.

19. A compound represented by the general formula (2), 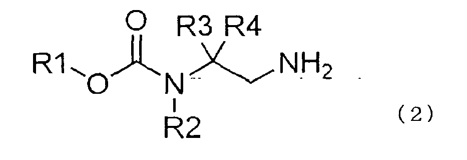 wherein, in the formula, R1 represents an alkyl group having 1 to 6 carbon atoms which is substituted with at least one fluorine atom or a cycloalkyl group having 3 to 6 carbon atoms which is substituted with at least one fluorine atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom).

20. The compound represented by the general formula (2) as set forth in claim 19, wherein, in the formula, R1 represents an alkyl group having 1 to 6 carbon atoms which is substituted with at least one fluorine atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom).

21. A compound represented by the general formula (1), 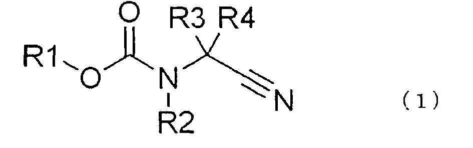 wherein, in the formula, R1 represents an alkyl group having 1 to 6 carbon atoms which is substituted with at least one fluorine atom or a cycloalkyl group having 3 to 6 carbon atoms which is substituted with at least one fluorine atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom).

22. The compound represented by the general formula (1) as set forth in claim 21, wherein, in the formula, R1 represents an alkyl group having 1 to 6 carbon atoms which is substituted with at least one fluorine atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom).

23. A compound represented by the general formula (5), 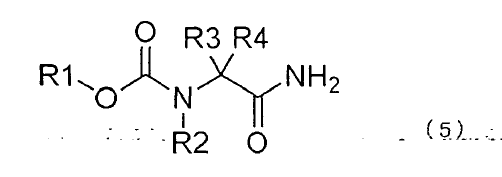 wherein, in the formula, R1 represents an alkyl group having 1 to 6 carbon atoms which is substituted with at least one fluorine atom or a cycloalkyl group having 3 to 6 carbon atoms which is substituted with at least one fluorine atom; R2 represents a hydrogen atom, an alkyl group having 1 to 6 carbon atoms, a cycloalkyl group having 3 to 6 carbon atoms, an aryl group which may be substituted or a heteroaryl group which may be substituted; R3 and R4 each independently represent a hydrogen atom, an alkyl group having 1 to 6 carbon atoms which may be substituted, a cycloalkyl group having 3 to 6 carbon atoms which may be substituted, an aryl group which may be substituted, an arylalkyl group which may be substituted, a heteroaryl group which may be substituted or a heteroarylalkyl group which may be substituted; and R3 and R4 may be bonded with each other to form a ring structure having 3 to 6 carbon atoms, or any one of R3 and R4 may be bonded with R2 to form a ring structure having 5 to 6 atoms in total (4 to 5 carbon atoms and 1 nitrogen atom).

24. The compound represented by the general formula (5) as set forth in claim 23, wherein, in the formula, R1 represents an alkyl group having 1 to 6 carbon atoms which is substituted with at least one fluorine atom; R2 represents a hydrogen atom or an alkyl group having 1 to 6 carbon atoms; and R3 and R4 each independently represent a hydrogen atom, an alkyl group having 1 to 6 carbon atoms which may be substituted, an aryl group which may be substituted or an arylalkyl group which may be substituted, and any one of R3 and R4 may be bonded with R2 to form a ring structure having 5 to 6 atoms in total (4 to 5 carbon atoms and 1 nitrogen atom).
